(19) **Europäisches Patentamt / European Patent Office / Office européen des brevets**

(11) **EP 4 706 543 A1**

(12) **EUROPEAN PATENT APPLICATION**
published in accordance with Art. 153(4) EPC

(43) Date of publication:
**11.03.2026 Bulletin 2026/11**

(21) Application number: **24811287.2**

(22) Date of filing: **26.04.2024**

(51) International Patent Classification (IPC):
*A61B 6/00* (2024.01)    *G16H 40/60* (2018.01)
*G06N 20/00* (2019.01)    *A61B 6/42* (2024.01)
*A61B 6/58* (2024.01)

(52) Cooperative Patent Classification (CPC):
**A61B 6/542; A61B 6/00; A61B 6/42; A61B 6/4283; A61B 6/4405; A61B 6/488; A61B 6/58; G06N 20/00; G16H 40/60;** A61B 6/4464

(86) International application number:
**PCT/KR2024/005700**

(87) International publication number:
**WO 2024/242348 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.05.2023 KR 20230065888**
**14.07.2023 KR 20230091999**

(71) Applicant: **Samsung Electronics Co., Ltd.**
**Suwon-si, Gyeonggi-do 16677 (KR)**

(72) Inventors:
• **LEE, Donghun**
  **Suwon-si, Gyeonggi-do 16677 (KR)**
• **KIM, Donghyuk**
  **Suwon-si, Gyeonggi-do 16677 (KR)**
• **KIM, Sanguk**
  **Suwon-si, Gyeonggi-do 16677 (KR)**
• **KIM, Youngik**
  **Suwon-si, Gyeonggi-do 16677 (KR)**

(74) Representative: **Walaski, Jan Filip et al**
**Venner Shipley LLP**
**200 Aldersgate**
**London EC1A 4HD (GB)**

(54) **X-RAY APPARATUS FOR PERFORMING AUTOMATIC EXPOSURE CONTROL, AND CONTROL METHOD THEREOF**

(57)     An X-ray apparatus may include an X-ray irradiation module for outputting an X-ray; an X-ray detector for detecting the X-ray output from the X-ray irradiation module; memory storing at least one instruction; and at least one processor configured to execute the at least one instruction, wherein the at least one processor may be configured to execute the at least one instruction to control the X-ray irradiation module to output an X-ray for a first exposure time to perform scout imaging, read out X-ray detection values from a plurality of sampling pixels corresponding to some of a plurality of pixels of the X-ray detector for the first exposure time to generate scout imaging data corresponding to the scout imaging, control the X-ray irradiation module to output an X-ray for a second exposure time determined based on the scout imaging data to perform main imaging, and read out X-ray detection values from the plurality of pixels of the X-ray detector for the second exposure time to generate a main imaging image.

**(Cont. next page)**

EP 4 706 543 A1

# FIG. 1

## Description

## Technical Field

An embodiment of the disclosure relates to an X-ray apparatus for performing auto-exposure control, a method of controlling the X-ray apparatus, and a computer-readable recording medium having a program recorded thereon to cause a computer to perform the method of controlling the X-ray apparatus.

## Background Art

An X-ray apparatus generates an X-ray image by taking an image of an object. With advancements in imaging automation, a stationary X-ray apparatus of a room DR type is equipped with various automation functions such as automatic source-detector alignment, auto-exposure control, etc.. With such automation functions, the X-ray apparatus may assist radiologists in their work and reduce deviations in the quality of captured images. On the other hand, unlike the stationary X-ray apparatus, a mobile X-ray apparatus of a mobile DR type is unable to use Bucky, a key element of automation, and thus has a problem using the automation functions such as the automatic source-detector alignment and the auto-exposure control.

## Disclosure of Invention

## Solution to Problem

According to an aspect of an embodiment of the disclosure, there may be provided an X-ray apparatus including an X-ray irradiation module configured to output an X-ray; an X-ray detector configured to detect the X-ray output from the X-ray irradiation module; memory storing at least one instruction; and at least one processor configured to execute the at least one instruction, wherein the at least one processor is configured to execute the at least one instruction to control the X-ray irradiation module to output an X-ray for a first exposure time to perform scout imaging, read out X-ray detection values from a plurality of sampling pixels corresponding to some of a plurality of pixels of the X-ray detector for the first exposure time to generate scout imaging data corresponding to the scout imaging, control the X-ray irradiation module to output an X-ray for a second exposure time determined based on the scout imaging data to perform main imaging, and read out X-ray detection values from the plurality of pixels of the X-ray detector for the second exposure time to generate a main imaging image.

According to an aspect of an embodiment of the disclosure, there may be provided a method of controlling an X-ray apparatus including performing scout imaging by outputting an X-ray for a first exposure time; reading out X-ray detection values from a plurality of sampling pixels corresponding to some of a plurality of pixels of the X-ray detector for the first exposure time to generate scout imaging data corresponding to the scout imaging; performing main imaging by outputting an X-ray for a second exposure time determined based on the scout imaging data; and reading out an X-ray detection value from the plurality of pixels of the X-ray detector for the second exposure time to generate a main imaging image.

According to an embodiment of the disclosure, provided is a computer-readable recording medium having a program recorded thereon to cause a computer to perform an X-ray apparatus control method.

## Brief Description of Drawings

FIG. 1 is an exterior view illustrating a configuration of an X-ray apparatus, according to an embodiment of the present disclosure.
FIG. 2 is an exterior view of a portable X-ray detector.
FIG. 3 is an exterior view of a mobile X-ray apparatus as an example of an X-ray apparatus.
FIG. 4 is a block diagram illustrating a configuration of an X-ray apparatus, according to an embodiment of the present disclosure.
FIG. 5 is a flowchart illustrating a method of controlling an X-ray apparatus, according to an embodiment of the present disclosure.
FIG. 6 illustrates a layout of sampling pixels, according to an embodiment of the present disclosure.
FIG. 7 is a graph representing relations between the number of sampling pixels, mapping accuracy of a region of interest, and a computation time, according to an embodiment of the present disclosure.
FIG. 8 is a diagram illustrating a procedure for performing scout imaging and main imaging, according to an embodiment of the present disclosure.
FIG. 9 is a flowchart illustrating operations of an X-ray irradiation module, an X-ray detector and a workstation, according to an embodiment of the present disclosure.
FIG. 10 is a block diagram illustrating a configuration of an X-ray apparatus, according to an embodiment of the present disclosure.
FIG. 11 is a flowchart illustrating operations of an X-ray irradiation module, an X-ray detector and an external device, according to an embodiment of the present disclosure.
FIG. 12 illustrates operation of an occasion when an X-ray detector and a workstation communicate with each other based on wireless communication, according to an embodiment of the present disclosure.
FIG. 13 is a block diagram illustrating a configuration of an X-ray apparatus, according to an embodiment of the present disclosure.
FIG. 14 is a diagram illustrating an example in which an X-ray apparatus determines a region of interest, according to an embodiment of the present disclosure.

FIG. 15 is a diagram illustrating a procedure for determining a region of interest by using a machine learning model, according to an embodiment of the present disclosure.

FIG. 16 is a diagram illustrating a procedure for training a machine learning model, according to an embodiment of the present disclosure.

FIG. 17 is a diagram illustrating a procedure for selecting a machine learning model depending on an imaging area, according to an embodiment of the present disclosure.

FIG. 18A is a diagram illustrating a procedure for setting a region of interest excluding an artificial device area, according to an embodiment of the present disclosure.

FIG. 18B is a diagram illustrating a procedure for setting a region of interest excluding an organ damage area, according to an embodiment of the present disclosure.

FIG. 19 is a diagram illustrating a case of setting a region of interest to a default area, according to an embodiment of the present disclosure.

FIG. 20 is a flowchart illustrating a procedure for switching to a manual imaging mode when a second exposure time exceeds a reference time, according to an embodiment of the present disclosure.

## Mode for the Invention

It is understood that various embodiments of the disclosure and associated terms are not intended to limit technical features herein to particular embodiments, but encompass various changes, equivalents, or substitutions.

Like reference numerals may be used for like or related elements throughout the drawings.

The singular form of a noun corresponding to an item may include one or more items unless the context states otherwise.

Throughout the specification, "A or B", "at least one of A and B", "at least one of A or B", "A, B or C", "at least one of A, B and C", and "at least one of A, B, or C" may each include any one or all the possible combinations of A, B and C.

The expression "and/or" is interpreted to include a combination or any of associated elements.

Terms like "first", "second", etc., may be simply used to distinguish an element from another, without limiting the elements in a certain sense (e.g., in terms of importance or order).

When an element is mentioned as being "coupled" or "connected" to another element with or without an adverb "functionally" or "operatively", it means that the element may be connected to the other element directly (e.g., by wire), wirelessly, or through a third element.

It will be further understood that the terms "comprise" and/or "comprising," when used in this specification, specify the presence of stated features, integers, steps, operations, elements, parts or combinations thereof, but do not preclude the possible presence or addition of one or more other features, integers, steps, operations, elements, components, and/or groups thereof.

When an element is mentioned as being "connected to", "coupled to", "supported on" or "contacting" another element, it includes not only a case that the elements are directly connected to, coupled to, supported on or contact each other but also a case that the elements are connected to, coupled to, supported on or contact each other through a third element.

Throughout the specification, when an element is mentioned as being located "on" another element, it implies not only that the element is abut on the other element but also that a third element exists between the two elements.

In the present disclosure, images may include a medical image obtained by an X-ray imaging apparatus.

In the disclosure, the term 'object' refers to a target to be imaged, including a human, an animal or a part thereof. For example, the object may include a part (e.g., organ) of the body or a phantom.

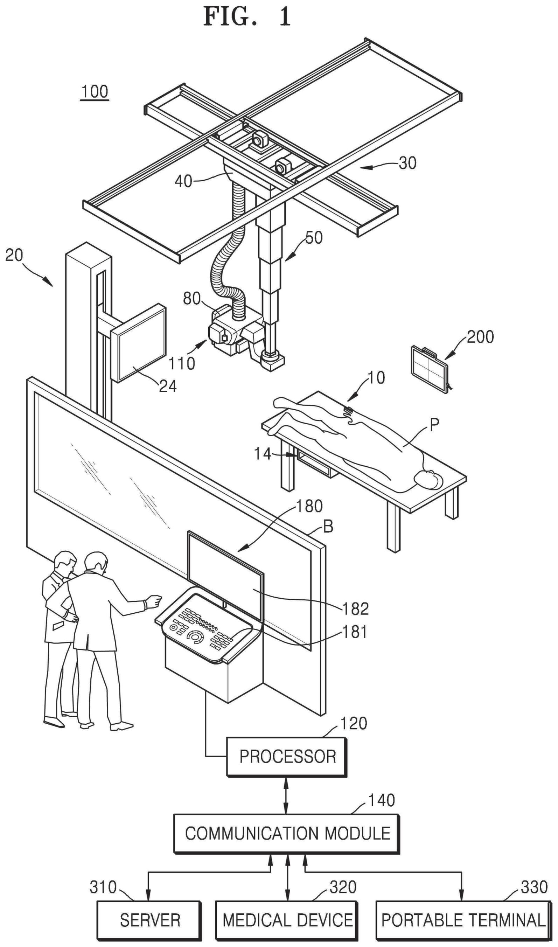

FIG. 1 is an exterior view illustrating a configuration of an X-ray apparatus, according to an embodiment of the present disclosure. In FIG. 1, described is a stationary X-ray apparatus as an example.

Referring to FIG. 1, an X-ray apparatus 100 includes an X-ray irradiation module 110 for generating and irradiating an X-ray, and an X-ray detector 200 for detecting the X-ray irradiated from the X-ray irradiation module 110 and penetrating the object. The X-ray apparatus 100 also includes a workstation 180 for receiving a command from the user and providing information. Furthermore, the X-ray apparatus 100 may include a processor 120 for controlling the X-ray apparatus 100 according to an input command, and a communication module 140 for communicating with an external device.

Part or all of the components of the processor 120 and the communication module 140 may be included in the workstation 180 or provided separately from the workstation 180.

The X-ray irradiation module 110 may be equipped with an X-ray source for generating an X-ray and a collimator for adjusting an irradiation area of the X-ray generated from the X-ray source.

A guide rail 30 may be installed on the ceiling of an examination room where the X-ray apparatus 100 is placed. The X-ray irradiation module 110 is connected to a mobile carriage 40 that moves along the guide rail 30. As the mobile carriage 40 is moved along the guide rail 30, the X-ray irradiation module 110 may be moved into a position corresponding to an object P. The mobile carriage 40 and the X-ray irradiation module 110 are connected through a foldable post frame 50. By adjusting the length of the post frame, the height of the X-ray irradiation may be adjusted.

An input interface 181 for receiving commands from the user and a display 182 for displaying information may be arranged on the workstation 180.

The input interface 181 may receive commands for controlling position of the X-ray irradiation module 110, imaging protocol, imaging conditions, imaging timing, etc. The input interface 181 may include a keyboard, a mouse, a touch screen, or a voice recognizer.

The display 182 may display a graphic user interface (GUI) view for guiding the user's input, an X-ray image, a GUI view that indicates a condition of the X-ray apparatus 100, etc.

The processor 120 may control imaging timing, imaging conditions, etc., of the X-ray irradiation module 110 according to a command input from the user. Furthermore, the processor 120 may generate an X-ray image based on image data received from the X-ray detector 200. The processor 120 may also control the location or posture of an installation part 14 or 24 where the X-ray irradiation module 110 or the X-ray detector 200 is installed, according to the imaging protocol and the position of the object P.

The processor 120 may include memory for storing a program for carrying out the aforementioned and following operations, and a processor for executing the program. The processor 120 may include a single processor or a plurality of processors, and in the latter case, the plurality of processors may be integrated in a single chip or may be physically separated.

The X-ray apparatus 100 may be connected to an external device (e.g., an external server 310, a medical device 320 or a portable terminal 330 (e.g., a smartphone, a tablet PC, a wearable device, etc.)) through the communication module 140 to transmit or receive data.

The communication module 140 may include one or more components that enable communication with the external device, and include, for example, at least one of a short-range communication module, a wired communication module, and a wireless communication module.

The communication module 140 may receive a control signal from the external device and may also send the received control signal to the processor 120 in order for the processor 120 to control the X-ray apparatus 100 according to the received control signal.

The processor 120 may also control the external device based on a control signal of the processor 120 by transmitting the control signal to the external device through the communication module 140. For example, the external device may process data of the external device according to the control signal of the processor 120 received through the communication module 140.

The communication module 140 may further include an internal communication module that enables communication between the components of the X-ray apparatus 100. A program to control the X-ray apparatus 100 may be installed in the external device, and the program may include instructions to perform part or all of the operations of the processor 120.

The program may be installed in the portable terminal 330 in advance, or the user of the portable terminal 330 may install the program by downloading the program from a server that provides an application. A recording medium that stores the program may be included in the server that provides the application.

The X-ray detector 200 may be implemented as a stationary X-ray detector fixed onto a stand 20 or a table 10, detachably equipped in the install part 14 or 24, or implemented as a portable X-ray detector available at any place. The portable X-ray detector may be implemented in a wired type or a wireless type depending on the data transmission method and the power supplying method.

The X-ray detector 200 may or may not be included as a component of the X-ray apparatus 100. In the latter case, the X-ray detector 200 may be registered in the X-ray apparatus 100 by the user. Furthermore, in both cases, the X-ray detector 200 may be connected to the processor 120 through the communication module 140 to receive a control signal or transmit image data.

A sub user interface 80 may be arranged on one side of the X-ray irradiation module 110 to provide information for the user and receive a command from the user, and may perform some or all of the functions to be performed by the input interface 181 and the display 182 of the workstation 180.

In a case that some or all of the components of the processor 120 and the communication module 140 are separately arranged from the workstation 180, part or all of the processor 120 and communication module 140 may be included in the sub user interface 80 arranged in the X-ray irradiation module 110.

Although FIG. 1 shows the stationary X-ray apparatus connected to the ceiling of an examination room, the X-ray apparatus 100 may include ones in various configurations such as a C-arm type X-ray apparatus, a mobile X-ray apparatus, etc., within a range that is obvious to those of ordinary skill in the art.

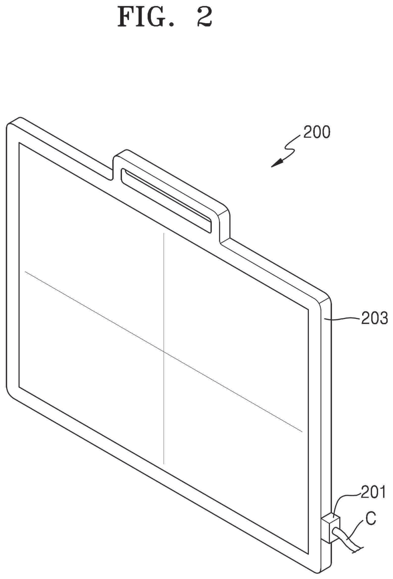

FIG. 2 is an exterior view of a portable X-ray detector.

As described above, the X-ray detector 200 used by the X-ray apparatus 100 may be implemented as a portable X-ray detector. In this case, the X-ray detector 200 may include a battery for supplying power and operate wirelessly. Furthermore, the X-ray detector 200, as shown in FIG. 2, may operate with a charging port 201 connected to a separate power supplier via a cable C.

Within a case 203 that defines an exterior of the X-ray detector 200, a detection element that detects an X-ray and converts it into image data, memory that temporarily or non-temporarily stores the image data, a communication module that receives a control signal from the X-ray apparatus 100 or transmits the image data to the X-ray apparatus 100, and a battery. Furthermore, the memory may store image correction information of the detector and unique identification information of the X-ray detector 200, and transmit the stored identification information while communicating with the X-ray apparatus 100.

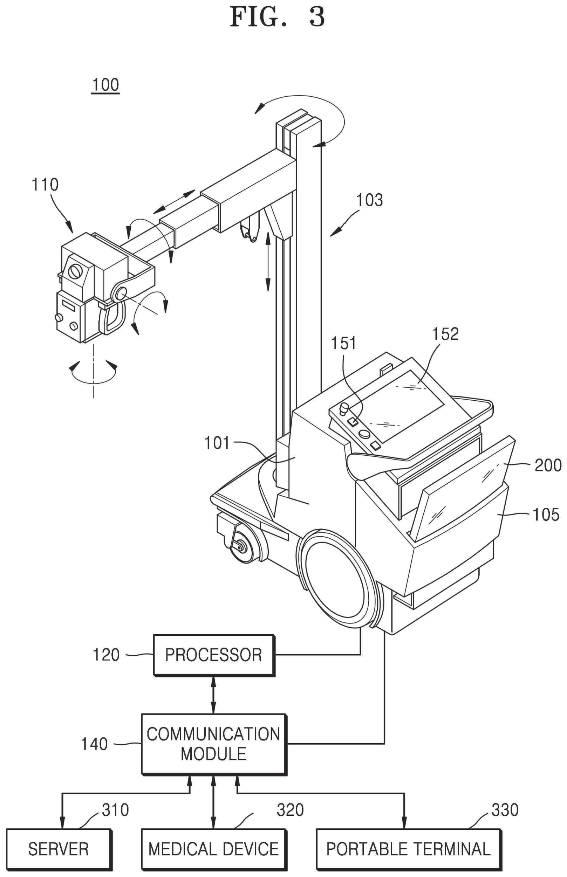

FIG. 3 is an exterior view of a mobile X-ray apparatus as an example of an X-ray apparatus.

Like reference numerals as in FIG. 1 indicate like functions, so descriptions thereof will not be repeated.

It is also possible that the X-ray apparatus 100 is implemented not only in the aforementioned ceiling type but also in a mobile type. In the case that the X-ray apparatus 100 is implemented as a mobile X-ray apparatus, as a main body 101 connected to the X-ray irradiation module 110 is freely movable and an arm 103 connecting the X-ray irradiation module 110 to the main body 101 is also able to rotate and move straight, the X-ray irradiation module 110 may be moved freely in three dimensional (3D) space.

A storage 105 for storing the X-ray detector 200 may be arranged on the main body 101. Furthermore, a charging terminal is arranged in the storage 105 to charge the X-ray detector 200 kept in the storage 105.

The input interface 151, the display 152, the processor 120 and the communication module 140 may be arranged in the main body 101. The image data obtained by the X-ray detector 200 may be transmitted to the main body 101, subjected to image processing, and displayed on the display 152 or transmitted to an external device through the communication module 140.

Furthermore, the processor 120 and the communication module 140 may be arranged separately from the main body 101, and it is also possible that only some of the components of the processor 120 and communication module 140 are arranged in the main body 101.

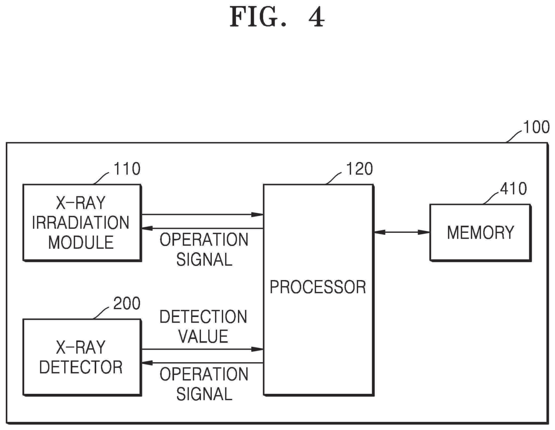

FIG. 4 is a block diagram illustrating a configuration of an X-ray apparatus, according to an embodiment of the present disclosure.

In an embodiment of the present disclosure, the X-ray apparatus 100 may include the X-ray irradiation module 110, the processor 120, the X-ray detector 200 and memory 410.

In an embodiment of the present disclosure, the X-ray apparatus 100 may correspond to a stationary X-ray apparatus or a mobile X-ray apparatus.

In an embodiment of the present disclosure, the X-ray apparatus 100 provides an auto-exposure control (AEC) operation. The AEC operation according to an embodiment of the disclosure will be described by comparing with an operation of an analog AEC device.

The analog AEC device includes an AEC chamber that includes an AEC sensor and an AEC circuit. The AEC chamber is installed in the Bucky of the stationary X-ray apparatus 100 to detect a dose of an X-ray emitted from the X-ray source in real time and output a cumulative dose value as a voltage. The voltage corresponding to the cumulative dose value may be input to the AEC circuit, and the AEC circuit may compare the detected dose with a preset dose and may cut off the output of the X-ray source when the cumulative dose value reaches the preset dose value. This operation may maintain image quality by making a constant X-ray dose reach the X-ray detector 200 despite the difference between patients and various imaging protocols.

A typical time for which X-rays are irradiated is on the order of 1 msec to 20 msec, and within this time range, the analog AEC device measures the X-ray dose in real time. The AEC circuit controls the output of the X-ray source after comparing the real-time X-ray dose with the set dose. For this, the AEC sensor and the AEC circuit are connected to the X-ray source by wires, and configured with analog circuits without using digital sampling techniques for prevention of processing time delay and circuit safety.

The analog AEC device converts a signal output from the AEC sensor to a voltage value in real time, amplifies the voltage value, and compares the voltage value with a reference voltage. This enables functional implementation with a simple circuit configuration. However, the analog AEC device needs to establish communication with no signal delay because the signal is transmitted in real time. For this, the connection of the communication is restricted to be configured with wires, making it difficult to use the AEC by wire/wirelessly in a mobile irradiation environment.

Furthermore, in a case of performing the AEC, when the X-ray source, the patient, the AEC sensor and the X-ray detector are misaligned with each other, it may cause overirradiation. In a case of a stationary DR (or room DR), a probability of the misalignment is low because the geometry of the devices is fixed. On the contrary, as it is not possible to fix the geometry of the devices in a case of using the AEC in a mobile environment, there is a limitation that the alignment is difficult.

In an embodiment of the present disclosure, the X-ray apparatus 100 has an effect of being able to stably detect an AEC area even when the X-ray source, the object and the X-ray detector are misaligned with each other.

Furthermore, in an embodiment of the present disclosure, the X-ray apparatus 100 has an effect of being able to actively establish a region of interest such that an area distorted because of an external device such as a pacemaker or a disease is excluded from the region of interest.

Also, in an embodiment of the present disclosure, the X-ray apparatus 100 calculates a main shot irradiation condition that considers characteristics of the X-ray detector, thereby having an effect of being able to obtain a uniform X-ray image for each X-ray detector.

Moreover, in an embodiment of the present disclosure, an AEC configuration corresponding to a signal latency caused by a transmission method is proposed, and with this, an AEC operation is activated wirelessly in a mobile environment, thereby facilitating enhancement of X-ray image quality.

In an embodiment of the present disclosure, the X-ray apparatus 100 performs scout imaging and main imaging. The scout imaging and the main imaging are sequentially performed in response to a one-time imaging signal. The X-ray apparatus 100 irradiates X-rays for a first exposure time to perform scout imaging, and after the scout imaging, irradiates X-rays for a second exposure time to perform main imaging. The second exposure time is determined based on scout imaging data generated by the scout imaging.

The X-ray irradiation module 110 may be equipped with an X-ray source for generating an X-ray and a collimator for adjusting an irradiation region of the X-ray generated by the X-ray source. The X-ray irradiation module 110 may include a high voltage generator (HVG) that generates a high voltage and outputs the high voltage to the X-ray source.

The X-ray irradiation module 110 may control whether to output an X-ray, an X-ray output intensity, an X-ray output time, etc., based on an operation signal received from the processor 120. The X-ray irradiation module 110 may control the X-ray output time based on an exposure time control signal received from the processor 120.

The X-ray detector 200 detects an X-ray output from the X-ray irradiation module 110 and having passed the object P. In an embodiment of the present disclosure, the X-ray detector 200 may correspond to a digital radiography (DR) detector. The X-ray detector 200 includes a plurality of pixels, and obtains an X-ray detection value by reading out a detection value from each pixel. Each pixel may include a scintillator and a photo diode, and obtain the X-ray detection value by performing photoelectric conversion on the incident X-ray.

The X-ray detector 200 includes a read-out circuit for reading out a detection value from each pixel. The X-ray detector 200 reads out the detection value from each pixel for a read-out interval and transmits the detection value to the processor 120. Furthermore, the processor 120 may generate an X-ray image based on the X-ray detection values received from the X-ray detector 200.

**In** an embodiment of the present disclosure, the X-ray detector 200 may read out X-ray detection values, for scout imaging, from a plurality of sampling pixels corresponding to some of the whole pixels. The X-ray detector 200 may also read out X-ray detection values from the whole pixels for main imaging. The X-ray detector 200 may read out X-ray detection values from sampling pixels or the whole pixels based on an operation signal of the processor 120.

The processor 120 controls general operation of the X-ray apparatus 100 and processes data. The processor 120 may include one or more processors. The processor 120 may execute instructions or commands stored in the memory 410 to perform a certain operation. Furthermore, the processor 120 controls operations of the components included in the X-ray apparatus 100. The processor 120 may include a central processing unit (CPU), a microprocessor, a neural processing unit (NPU), etc.

The processor 120 may control the X-ray irradiation module 110. The processor 120 may control X-ray output timing, an X-ray output time, an X-ray intensity, etc., of the X-ray irradiation module 110. The processor 120 may also control the output current, output voltage or output charge quantity of the HVG of the X-ray irradiation module 110.

The processor 120 also controls the X-ray detector 200 and receives the X-ray detection value from the X-ray detector 200.

The processor 120 may control the X-ray detector 200 to perform a refresh operation to initialize the detection value of each pixel of the X-ray detector 200. The processor 120 generates a refresh operation signal to initialize the detection value of each pixel and outputs the refresh operation signal to the X-ray detector 200 so that the X-ray detector 200 performs the refresh operation.

The processor 120 also outputs a read-out operation signal to the X-ray detector 200 for the X-ray detector 200 to read out X-ray detection values from some or the whole pixels. The processor 120 generates and outputs, to the X-ray detector 200, a read-out operation signal to read out X-ray detection values from sampling pixels corresponding to some pixels of the X-ray detector 200, while performing the scout imaging. Furthermore, the processor 120 generates and outputs, to the X-ray detector 200, a read-out operation signal to read out X-ray detection values from the whole pixels of the X-ray detector 200, while performing the main imaging.

Furthermore, the processor 120 generates an X-ray image based on the X-ray detection values received from the X-ray detector 200. The processor 120 generates scout imaging data from the X-ray detection values obtained by the scout imaging. The scout imaging data may correspond to, for example, an image or a data set of the X-ray detection values obtained by the scout imaging, but is not limited thereto.

The processor 120 generates a main imaging image from the X-ray detection values obtained by the main imaging. The processor 120 may transmit the scout imaging data or the main imaging image to a workstation, a server or an external device. Furthermore, the processor 120 may store the scout imaging data or the main imaging image in the memory 410.

Moreover, the processor 120 controls the X-ray irradiation module 110 to output an X-ray for the first exposure time to perform scout imaging. The processor 120 computes a second exposure time for main imaging based on the scout imaging data. It is also possible that the computing of the second exposure time is performed by an external device. The processor 120 controls the X-ray irradiation module 110 to output an X-ray for the second exposure time to perform main imaging.

The memory 410 stores various information, data, instructions, programs, etc., required for operation of the X-ray apparatus 100. The memory 410 may include at least one of a volatile memory or a non-volatile memory or a combination thereof. The memory 410 may include at least one type of storage medium including a flash memory, a hard disk, a multimedia card micro type memory, a card type memory (e.g., SD or XD memory), a random access memory (RAM), a static RAM (SRAM), a read-only memory (ROM), an electrically erasable programmable ROM (EEPROM), a programmable ROM (PROM), a magnetic memory, a magnetic disk, and an optical disk. The memory 410 may correspond to a web storage or a cloud server that performs a storage function on the Internet.

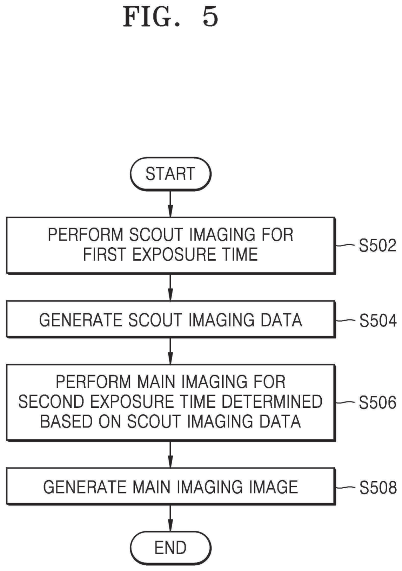

FIG. 5 is a flowchart illustrating a method of controlling an X-ray apparatus, according to an embodiment of the present disclosure.

Respective operations of the method of controlling the X-ray apparatus of the present disclosure may be performed by various types of X-ray apparatuses. The disclosure is focused on an embodiment in which the X-ray apparatus 100 according to embodiments of the disclosure performs a method of controlling the X-ray apparatus. Accordingly, an embodiment described of the X-ray apparatus 100 is applicable to an embodiment of the method of controlling the X-ray apparatus, and vice versa. An embodiment of the method of controlling the X-ray apparatus is not limited to being performed by the X-ray apparatus 100 as disclosed herein, but may be performed by various types of X-ray apparatuses.

In operation S502, the X-ray apparatus 100 outputs an X-ray for the first exposure time to perform scout imaging. The first exposure time is a preset exposure time for scout imaging. The X-ray apparatus 100 may control the X-ray irradiation module to output an X-ray for the first exposure time. The X-ray apparatus 100 may perform scout imaging based on an imaging control signal input by the user. The X-ray detector 200 may be refreshed before scout imaging, and may accumulate the X-rays while the scout imaging is being performed.

The X-ray apparatus 100 may sequentially perform the scout imaging and main imaging when an imaging control signal is input by the user and an AEC mode is activated. When the AEC mode is not activated, the X-ray apparatus 100 may perform main imaging without performing scout imaging.

Subsequently, in operation S504, the X-ray apparatus 100 generates scout imaging data from the X-ray detection values generated by the X-ray detector 200 according to the scout imaging. In an embodiment of the disclosure, the X-ray apparatus 100 reads out X-ray detection values from sampling pixels corresponding to some pixels of the X-ray detector 200 that performs scout imaging, and generates scout imaging data from the read-out X-ray detection values.

Subsequently, in operation S506, the X-ray apparatus 100 performs main imaging for the second exposure time determined based on the scout imaging data.

The X-ray apparatus 100 determines the second exposure time for main imaging based on the scout imaging data. The X-ray apparatus 100 determines the second exposure time required for the X-ray dose in a region of interest of the scout imaging data to reach a target dose, based on the scout imaging data. The X-ray apparatus 100 may compute a cumulative dose for the first exposure time in the region of interest based on pixel values of the region of interest of the scout imaging data, and determine the second exposure time based on the cumulative dose. The X-ray apparatus 100 may determine the second exposure time based on the cumulative dose and attributes of the X-ray detector 200.

In an embodiment of the disclosure, the second exposure time may be computed by an external device that communicates with the X-ray apparatus 100. The X-ray apparatus 100 may transmit the scout imaging data to the external device and receive the second exposure time from the external device.

The X-ray apparatus 100 irradiates an X-ray for the determined second exposure time to perform main imaging. The X-ray apparatus 100 outputs an X-ray from the X-ray irradiation module 110 for the second exposure time. The X-ray detector 200 may be refreshed after the scout imaging, and may accumulate the X-ray after the main imaging is initiated.

Next, in operation S508, the X-ray apparatus 100 generates a main imaging image. The X-ray apparatus 100 reads out X-ray detection values from the pixels of the X-ray detector 200 after a lapse of the second exposure time, and generates the main imaging image from the read-out X-ray detection values. The main imaging image may be generated based on more X-ray detection values of the pixels than the scout imaging data.

In an embodiment of the present disclosure, the X-ray apparatus 100 may have an AEC mode to perform an AEC operation. The AEC mode may be activated or inactivated based on the user input. When the AEC mode is activated, the X-ray apparatus 100 performs an AEC operation that sequentially performs scout imaging and main imaging. When the AEC mode is not activated, the X-ray apparatus 100 determines an X-ray exposure time based on a user input. For example, when the AEC mode is not activated, the X-ray apparatus 100 operates in a manual mode and may irradiate an X-ray while the user is pressing a hand switch to control the X-ray output.

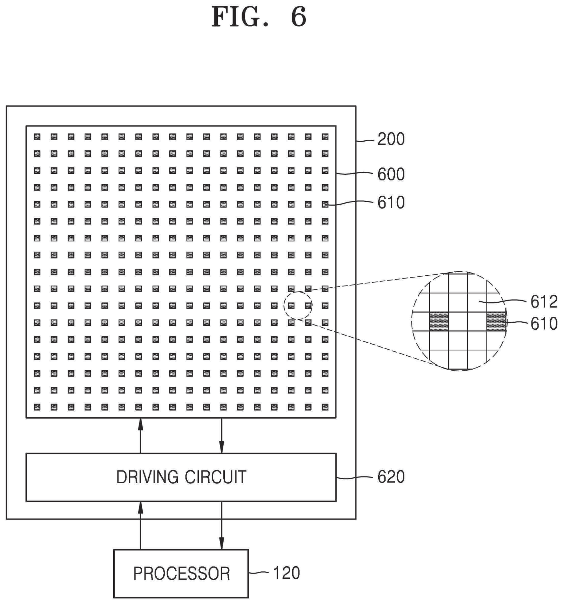

FIG. 6 illustrates a layout of sampling pixels, according to an embodiment of the present disclosure.

The X-ray apparatus 100 reads out X-ray detection values from sampling pixels corresponding to some of the whole pixels of the X-ray detector 200 while performing scout imaging.

The X-ray detector 200 may include a pixel array 600 and a driving circuit 620. The pixel array 600 includes a plurality of pixels. The driving circuit 620 drives the pixel array 600 and reads out X-ray detection values from the pixel array 600. The driving circuit 620 may operate according to a driving signal input from the processor 120.

The pixel array 600 is arranged in the form of a 2D matrix. The pixel array 600 includes a plurality of pixels 612. In an embodiment of the present disclosure, some of the plurality of pixels 612 correspond to sampling pixels 610. The sampling pixels 610 correspond to some of the plurality of pixels 612. For example, the sampling pixels 610 may be selected one out of three in horizontal and vertical directions. The number and pattern of the sampling pixels 610 may be selected in various ways.

The driving circuit 620 reads out X-ray detection values from the sampling pixels 610 when scout imaging is performed. The driving circuit 620 selects a pixel corresponding to the sampling pixel 610 and reads out an X-ray detection value therefrom. In the case of performing scout imaging, the driving circuit 620 does not read out X-ray detection values from other pixels than the sampling pixels 610. The driving circuit 620 outputs the read-out pixel values of the sampling pixels 610 to the processor 120. The processor 120 generates scout imaging data from the X-ray detection values of the sampling pixels 610.

The driving circuit 620 reads out X-ray detection values from the whole pixels 612 of the pixel array 600 when main imaging is performed. In the case of performing the main imaging, the driving circuit 620 may read out X-ray detection values from the sampling pixels 610 and the other pixels 612 than the sampling pixels 610. The driving circuit 620 outputs the read-out pixel values of the whole pixels 612 to the processor 120. The processor 120 generates a main imaging image from the X-ray detection values of the whole pixels 612.

In an embodiment of the disclosure, the X-ray detector 200 may include an extra sensor for detecting an X-ray during the scout imaging. The extra sensor may correspond to some pixels in, for example, an ion chamber sensor or the X-ray detector 200. The X-ray apparatus 100 may obtain sensor detection values from the extra sensor, and generate scout imaging data from the sensor detection values.

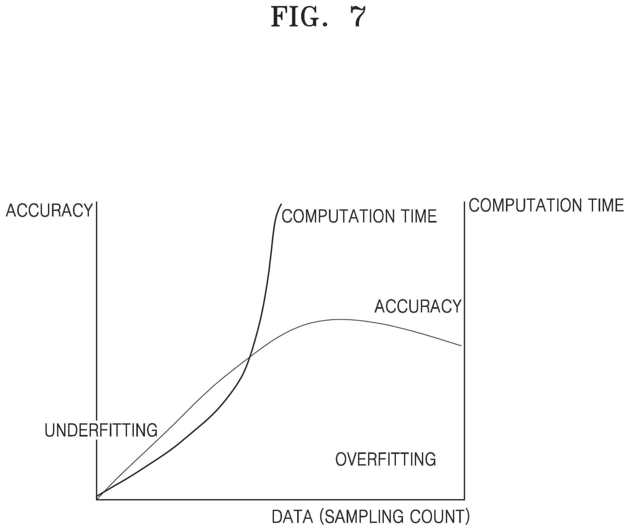

FIG. 7 is a graph representing relations between the number of sampling pixels, mapping accuracy of a region of interest, and a computation time, according to an embodiment of the present disclosure.

In an embodiment of the present disclosure, the mapping accuracy of a region of interest may vary depending on the number of sampling pixels.

The X-ray apparatus 100 determines a region of interest based on the scout imaging data, and determines the second exposure time for main imaging based on the X-ray detection values of the region of interest. When the number of sampling pixels 610 is reduced, the scout image resolution is lowered, so the mapping accuracy that represents whether the region of interest has been accurately defined may be reduced. On the other hand, the more the number of sampling pixels 610, the higher the mapping accuracy. However, when the number of sampling pixels 610 exceeds a certain number, the mapping accuracy is saturated at a certain level and does not increase even with an increase in number of sampling pixels 610.

Meanwhile, as the number of sampling pixels 610 increases, the computation time to generate scout imaging data increases. When the number of sampling pixels 610 increases, a read-out time for reading out X-ray detection values, a time for generating scout imaging data from the X-ray detection values, and a processing time for determining the second exposure time from the scout imaging data may increase.

In an embodiment of the present disclosure, the X-ray apparatus 100 may obtain relationship information between the number of sampling pixels 610, computation time and mapping accuracy, and determine the number of sampling pixels 610 based on a target computation time range and mapping accuracy range. The relationship information between the number of sampling pixels 610 of the X-ray apparatus 100, the computation time and the mapping accuracy may be predefined and stored in the memory 410. The number of the sampling pixels 610 may be determined in a product design procedure. In an embodiment of the disclosure, the mapping accuracy range or computation time range desired by the user may be input as a user input, and based on the user input, the number of sampling pixels 610 may be determined.

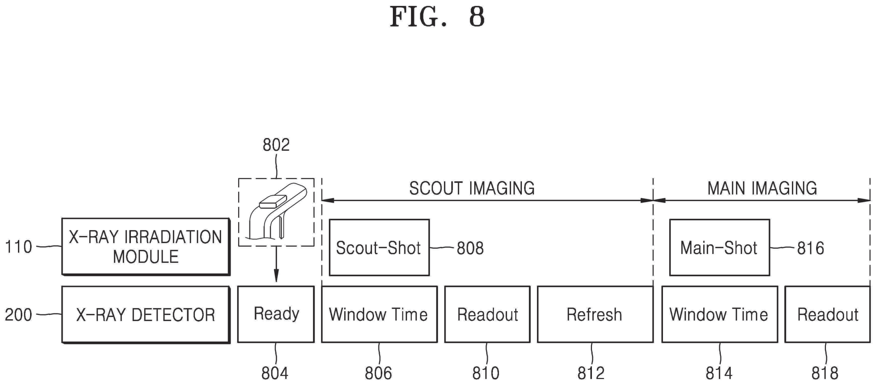

FIG. 8 is a diagram illustrating a procedure for performing scout imaging and main imaging, according to an embodiment of the present disclosure.

**In** an embodiment of the disclosure, the X-ray apparatus 100 sequentially performs scout imaging and main imaging based on an imaging control signal when the AEC mode is activated. While the scout imaging and the main imaging are being performed, the X-ray irradiation module 110 and the X-ray detector 200 operate in a certain sequence. In FIG. 8, operation timing of the X-ray irradiation module 110 and the X-ray detector 200 of the X-ray imaging apparatus 100 is described.

**In** operation 802, an imaging control signal is input from a hand switch of the X-ray apparatus 100. The imaging control signal is a control signal to request the X-ray apparatus 100 for X-ray imaging by the user.

In operation 804, the X-ray detector 200 performs an initial setting for an X-ray imaging operation in response to the imaging control signal. The X-ray detector 200 may initialize the status of the plurality of pixels 612, and apply an initial voltage to the plurality of pixels 612 and the driving circuit 620.

When the initial setting of the X-ray detector 200 is finished, the X-ray apparatus 100 performs a scout imaging operation. The scout imaging operation includes operations 806, 808 and 812.

In operation 806, the X-ray detector 200 performs X-ray detection for a first window time for scout imaging. For the first window time, the X-ray detector 200 generates an electric detection signal from X-rays entering the plurality of pixels 612. For the first window time, charges generated by the X-rays may be accumulated on the plurality of pixels 612. The voltage to the plurality of pixels 612 may vary depending on the detected cumulative X-ray doses.

In operation 808, the X-ray irradiation module 110 performs X-ray irradiation for scout imaging for the first exposure time in a time interval within the first window time. The X-ray irradiation module 110 may output a scout shot for the first exposure time. The first exposure time is set to be shorter than the first window time. The first exposure time may be set in advance.

When the first window time is over, the X-ray detector 200 reads out X-ray detection values from the sampling pixels 610 in operation 810. The driving circuit 620 of the X-ray detector 200 reads out the X-ray detection values from the sampling pixels 610 corresponding to some of the plurality of pixels 612. The X-ray detector 200 transmits the read-out X-ray detection values to the processor 120 or the memory 410.

When the read-out interval of scout imaging is over, the X-ray detector 200 refreshes the plurality of pixels 612 in operation 812. In an embodiment of the disclosure, the X-ray detector 200 may refresh the whole pixels 612 in operation 812. The X-ray detector 200 may refresh the X-ray detector 200 as the voltage level at each node of the plurality of pixels 612 is initialized to a certain level.

In an embodiment of the present disclosure, the X-ray apparatus 100 may generate scout imaging data based on the X-ray detection values during the refreshing interval of operation 812. Furthermore, the X-ray apparatus 100 may set the second exposure time for main imaging based on the scout imaging data during the refreshing interval. The X-ray apparatus 10 may set the X-ray irradiation module 110 for main imaging by using the set second exposure time and initial setting values (e.g., output voltage, output current, output charge quantity, etc.) for the X-ray irradiation module 110. For example, for main imaging, the X-ray apparatus 100 may set at least one of the second window time, the exposure time, the output voltage, the output current or the output charge quantity of the HVG of the X-ray irradiation module 110.

When the refreshing of the X-ray detector 200 is finished, the X-ray apparatus 100 performs a main imaging operation. The main imaging operation includes operations 814, 816 and 818.

In operation 814, the X-ray detector 200 performs X-ray detection for a second window time for main imaging. For the second window time, the X-ray detector 200 generates an electric detection signal from X-rays entering the plurality of pixels 612. For the second window time, charges generated by the X-rays may be accumulated on the plurality of pixels 612. The voltage to the plurality of pixels 612 may vary depending on the detected cumulative X-ray doses.

In operation 816, the X-ray irradiation module 110 performs X-ray irradiation for scout imaging for the second exposure time in a time interval within the second window time. The X-ray irradiation module 110 may output a scout shot for the second exposure time. The second exposure time is set to be shorter than the second window time. The second exposure time is determined based on scout imaging data generated by the scout imaging.

When the second window time is over, the X-ray detector 200 reads out X-ray detection values from the whole pixels 612 in operation 818. The driving circuit 620 of the X-ray detector 200 reads out the X-ray detection values from the plurality of pixels 612. The X-ray detector 200 transmits the read-out X-ray detection values to the processor 120 or the memory 410.

The X-ray apparatus 100 generates a main imaging image based on the read-out X-ray detection values in operation 818. The main imaging image is an output image corresponding to the imaging control signal of operation 802. The main imaging image may be displayed by the X-ray apparatus 100 or transmitted to an external device.

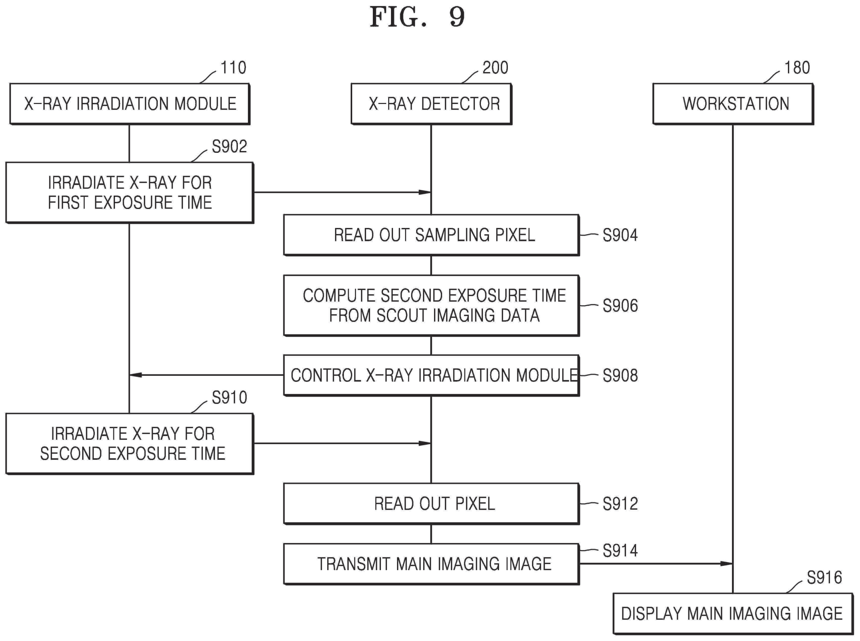

FIG. 9 is a flowchart illustrating operations of an X-ray irradiation module, an X-ray detector and a workstation, according to an embodiment of the present disclosure.

In an embodiment of the disclosure, the X-ray detector 200 may perform an operation of computing the second exposure time from the scout imaging data.

First, in operation S902, the X-ray irradiation module 110 irradiates X-rays for the first exposure time. The X-ray irradiation module 110 may irradiate X-rays during a time interval within the first window time interval of the X-ray detector 200.

Next, in operation S904, the X-ray detector 200 reads out X-ray detection values from the sampling pixels. The X-ray detector 200 generates scout imaging data from the read-out X-ray detection values.

Next, in operation S906, the X-ray detector 200 computes the second exposure time for main imaging based on the scout imaging data. The X-ray detector 200 may set a region of interest based on the scout imaging data, and compute the second exposure time based on the X-ray detection values of the region of interest. The X-ray detector 200 may compute the second exposure time based on a target X-ray detection value for the region of interest, X-ray detection values in the scout imaging data and attributes of the X-ray detector 200.

The X-ray detector 200 may set the target X-ray detection value based on an imaging protocol or imaging area information. Furthermore, the X-ray detector 200 may set the target X-ray detection value based on medical institute information or user settings.

Next, in operation S908, the X-ray detector 200 sets the X-ray irradiation module 110 before the main imaging is performed. The X-ray detector 200 controls the X-ray irradiation module 110 to irradiate an X-ray for the second exposure time. The X-ray detector 200 may also control the output voltage and output current of the HVG of the X-ray irradiation module 110.

Next, in operation S910, the X-ray irradiation module 100 irradiates X-rays for the second exposure time. The X-ray irradiation module 110 irradiates an X-ray with setting values set by the X-ray detector 200. The X-ray irradiation module 110 may output an X-ray in sync with the second window time of the X-ray detector 200 based on a control signal from the X-ray detector 200. The X-ray irradiation module 110 may irradiate an X-ray for the second exposure time within a time interval of the second window time of the X-ray detector 200. The X-ray detector 200 generates X-ray detection values from the plurality of pixels 612 while the X-ray irradiation module 110 is irradiating an X-ray for the second exposure time.

Next, in operation S912, the X-ray detector 200 reads out the X-ray detection values from the plurality of pixels 612 after the second window time is over. The X-ray detector 200 generates a main imaging image based on the read-out X-ray detection values.

Next, in operation S914, the X-ray detector 200 transmits the main imaging image to the workstation 180. The workstation 180 may correspond to a PC or user interface device included in the X-ray apparatus 100, or an external device that communicates with the X-ray apparatus 100.

Next, in operation S916, the workstation 180 displays the main imaging image. The workstation 180 may display the main imaging image along with various types of graphic user interfaces (GUIs). In an embodiment of the disclosure, the workstation 180 may receive scout imaging data from the X-ray detector 200 and display the scout imaging data.

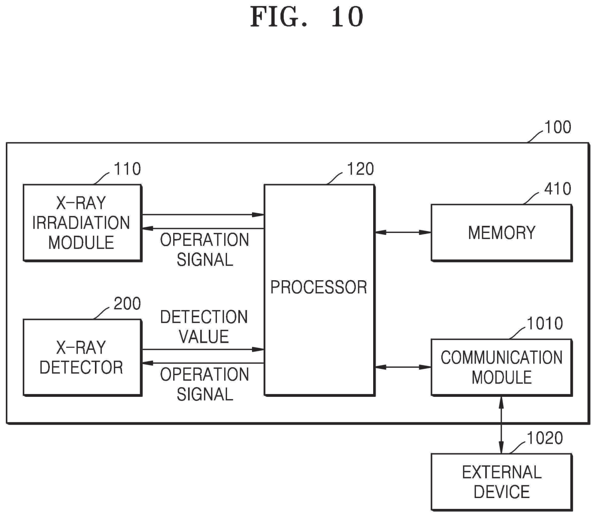

FIG. 10 is a block diagram illustrating a configuration of an X-ray apparatus, according to an embodiment of the present disclosure.

In an embodiment of the present disclosure, the X-ray apparatus 100 may include the X-ray irradiation module 110, the processor 120, the X-ray detector 200, the memory 410 and a communication module 1010. In FIG. 10, focused is a difference from the embodiment of FIG. 4.

The X-ray apparatus 100 communicates with an external device 1020 through the communication module 1010. The external device 1020 may correspond to a workstation, a tablet PC, a mobile phone, a desktop PC, a laptop PC, a server, a mobile medical device, a flexible device, etc.

The communication module 1010 may communicate with the external device by wire or wirelessly. In an embodiment of the present disclosure, the communication module 1010 may perform short-range communication, and may use, for example, Bluetooth, Bluetooth low energy (BLE), near field communication (NFC), WLAN (Wi-Fi), Zigbee, infrared data association (IrDA) communi-

nication, Wi-Fi direct (WFD), ultra wideband (UWB), Ant+ communication, etc. In an embodiment of the present disclosure, the communication module 1010 may use mobile communication and transmit or receive a wireless signal to or from at least one of a base station, an external terminal or a server over a mobile communication network.

Furthermore, the communication module 1010 may exchange data with a hospital server or another medical device in the hospital connected through a picture archiving and communication system (PACS). Moreover, the communication module 1010 may perform data communication according to the digital imaging and communications in medicine (DICOM) standard.

In an embodiment of the present disclosure, the X-ray apparatus 100 may transmit the scout imaging data and the main imaging image to the external device 1020 through the communication module 1010. Furthermore, the X-ray apparatus 100 may receive the second exposure time from the external device 1020 through the communication module 1010. The X-ray apparatus 100 transmits the scout imaging data to the external device 1020 through the communication module 1010, and the external device 1020 computes the second exposure time based on the scout imaging data. The external device 1020 transmits the computed second exposure time to the X-ray apparatus 100.

In an embodiment of the present disclosure, the communication module 1010 may correspond to the communication module 140 shown in FIG. 1.

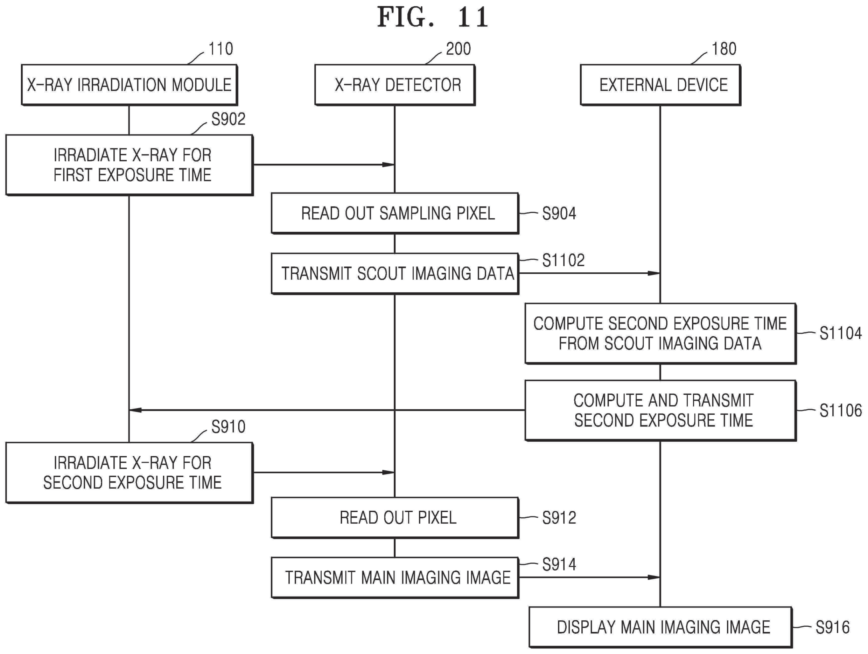

FIG. 11 is a flowchart illustrating operations of an X-ray irradiation module, an X-ray detector and an external device, according to an embodiment of the present disclosure.

In an embodiment of the present disclosure, the second exposure time is computed by the external device 1020. The external device 1020 receives the scout imaging data from the X-ray detector 200 and computes the second exposure time based on the scout imaging data. The external device 1020 transmits the second exposure time to the X-ray detector 200.

In an embodiment of the present disclosure, the external device 1020 may correspond to the workstation 180. The embodiment of FIG. 11 includes both an embodiment in which the workstation 180 computes the second exposure time from the scout imaging data and transmits the second exposure time to the X-ray irradiation module 110 and an embodiment in which the external device 1020 (e.g., a tablet PC, a smartphone, a PC, etc.) computes the second exposure time from the scout imaging data and transmits the second exposure time to the X-ray irradiation module 110.

First, in operation S902, the X-ray irradiation module 110 irradiates X-rays for the first exposure time. The X-ray irradiation module 110 may irradiate X-rays during a time interval within the first window time interval of the X-ray detector 200.

Next, in operation S904, the X-ray detector 200 reads out X-ray detection values from the sampling pixels. The X-ray detector 200 generates scout imaging data from the read-out X-ray detection values.

Next, in operation S1102, the X-ray detector 200 transmits the scout imaging data to the external device 1020. The X-ray detector 200 may establish communication with the external device 1020. The X-ray detector 200 transmits the scout imaging data to the external device 1020 that has undergone a certain authentication procedure. The external device 1020 receives the scout imaging data from the X-ray detector 200.

Next, in operation S1104, the external device 1020 computes the second exposure time for main imaging based on the scout imaging data. The external device 1020 may set a region of interest based on the scout imaging data, and compute the second exposure time based on the X-ray detection values of the region of interest. The external device 1020 may compute the second exposure time based on a target X-ray detection value for the region of interest, X-ray detection values in the scout imaging data and attributes of the X-ray detector 200. The X-ray detector 200 receives the second exposure time from the external device 1020.

The external device 1020 may set the target X-ray detection value based on an imaging protocol or imaging area information. Furthermore, the external device 1020 may set the target X-ray detection value based on medical institute information or user settings. The external device 1020 may receive attributes information of the X-ray detector 200 from the X-ray detector 200 or a server.

Next, in operation S1106, the X-ray detector 200 transmits the second exposure to the X-ray irradiation module 110 before main imaging is performed, and sets the X-ray irradiation module 110. The X-ray detector 200 controls the X-ray irradiation module 110 to irradiate an X-ray for the second exposure time. The X-ray detector 200 may also control the output voltage and output current of the HVG of the X-ray irradiation module 110.

Next, in operation S910, the X-ray irradiation module 100 irradiates X-rays for the second exposure time. The X-ray irradiation module 110 irradiates X-rays with setting values set by the X-ray detector 200. The X-ray irradiation module 110 may output X-rays in sync with the second window time of the X-ray detector 200 based on a control signal from the X-ray detector 200. The X-ray irradiation module 110 may irradiate X-rays for the second exposure time within a time interval of the second window time of the X-ray detector 200. The X-ray detector 200 generates X-ray detection values from the plurality of pixels 612 while the X-ray irradiation module 110 is irradiating X-rays for the second exposure time.

Next, in operation S912, the X-ray detector 200 reads out the X-ray detection values from the plurality of pixels 612 after the second window time is over. The X-ray detector 200 generates a main imaging image based on the read-out X-ray detection values.

Next, in operation S914, the X-ray detector 200 transmits the main imaging image to the external device 1020.

Subsequently, in operation S1106, the external device 1020 displays the main imaging image. The external device 1020 may display the main imaging image along with various types of graphic user interfaces (GUIs). In an embodiment of the disclosure, the external device 1020 may receive scout imaging data from the X-ray detector 200 and display the scout imaging data.

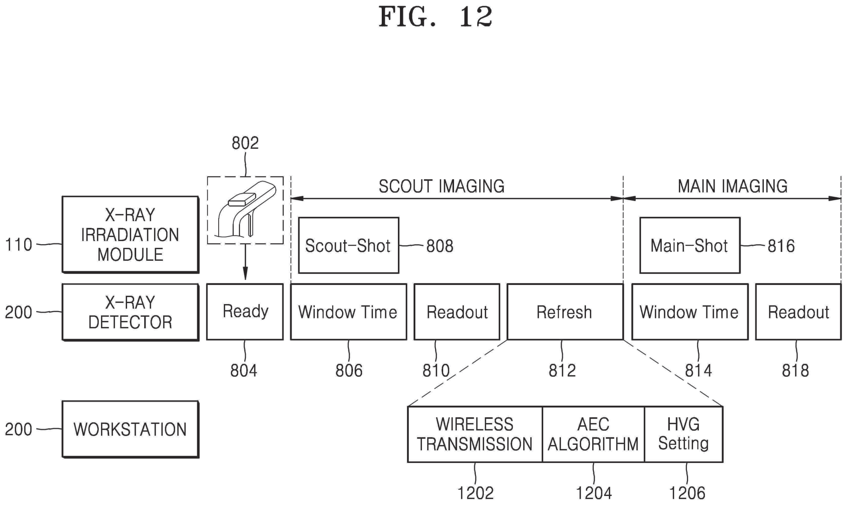

FIG. 12 illustrates operation of an occasion when an X-ray detector and a workstation communicate with each other based on wireless communication, according to an embodiment of the present disclosure.

In FIG. 12, descriptions overlapping those of FIG. 8 are not repeated, and focused is a difference from the embodiment of FIG. 8.

In an embodiment of the present disclosure, the X-ray detector 200 performs operations 806, 808, 810 and 812 for scout imaging. The X-ray detector 200 reads out X-ray detection values from the sampling pixels 610 in operation 810, and refreshes the plurality of pixels 612 in operation 812. In an embodiment of the present disclosure, the X-ray detector 200 wirelessly communicates with the workstation 180 and performs an operation of setting the HVG during the refreshing interval of operation 812. An example in which the workstation 180 performs operations 1202, 1204 and 1206 is shown in FIG. 12, but in an embodiment of the disclosure, it is also possible for the external device 1020, apart from the workstation 180, to perform operations 1202, 1204 and 1206.

In an embodiment of the present disclosure, the X-ray detector 200 transmits scout imaging data to the workstation 180 wirelessly in operation 1202 during the refreshing interval of operation 812. The workstation 180 receives the scout imaging data transmitted from the X-ray detector 200. The wireless transmission is slower than the wired transmission, having a certain delay. Hence, the existing AEC mode that requires real-time transmission has hard time using the wireless transmission. In an embodiment of the present disclosure, as the X-ray detector 200 of a digital type is used, communication is made with the workstation 180 wirelessly.

In operation S1204, the workstation 180 computes the second exposure time for main imaging based on the received scout imaging data. The workstation 180 determines the second exposure time based on the scout imaging data by performing an AEC algorithm.

Subsequently, in operation 1206, the workstation 180 sets the HVG of the X-ray irradiation module 110 based on the determined second exposure time. The workstation 180 may set the exposure time of the HVG to the second exposure time, and set the output current and output voltage of the HVG.

The X-ray irradiation module 110 and the X-ray detector 200 perform a main imaging operation when the settings of the HVG is completed by the workstation 180.

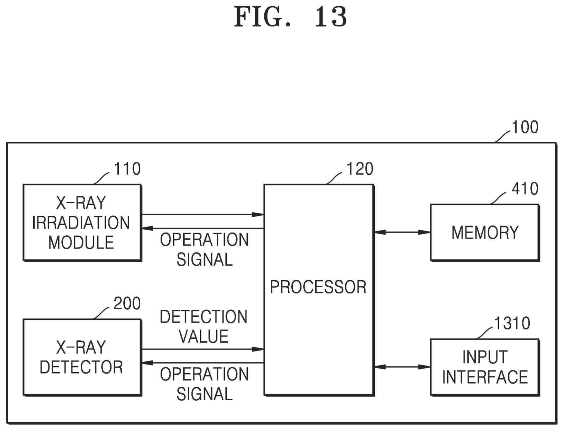

FIG. 13 is a block diagram illustrating a configuration of an X-ray apparatus, according to an embodiment of the present disclosure.

In an embodiment of the disclosure, the X-ray apparatus 100 may include the X-ray irradiation module 110, the processor 120, the X-ray detector 200, the memory 410 and an input interface 1310. In FIG. 13, focused is a difference from the embodiment of FIG. 4.

The input interface 1310 is an interface for receiving user inputs. The input interface 1310 may receive control commands or data from the user or an external device. The input interface 1310 may include, for example, a touch screen, a touch pad, a mouse, a keyboard, keys, buttons, a wheel, a jog or a dial.

In an embodiment of the present disclosure, the input interface 1310 may be equipped in the workstation 180. Furthermore, in an embodiment of the present disclosure, the input interface 1310 may be equipped in the X-ray irradiation module 110. In an embodiment of the present disclosure, the X-ray apparatus 100 may have a mobile X-ray apparatus type, and the input interface 1310 may be equipped in the main body of the mobile X-ray apparatus. For example, the input interface 1310 may correspond to the input interface 151 of FIG. 3.

In an embodiment of the present disclosure, the X-ray apparatus 100 receives a user input to select an imaging protocol through the input interface 1310. Furthermore, in an embodiment of the present disclosure, received is a user input to select an imaging area of the object.

The processor 120 may receive the user input to select the imaging protocol or imaging area through the input interface 1310 before the X-ray imaging is performed. The processor 120 may provide a GUI to select an imaging protocol or imaging area for the X-ray apparatus 100 to perform imaging, and receive, through the input interface 1310, a user input to select the imaging protocol or imaging area through the GUI.

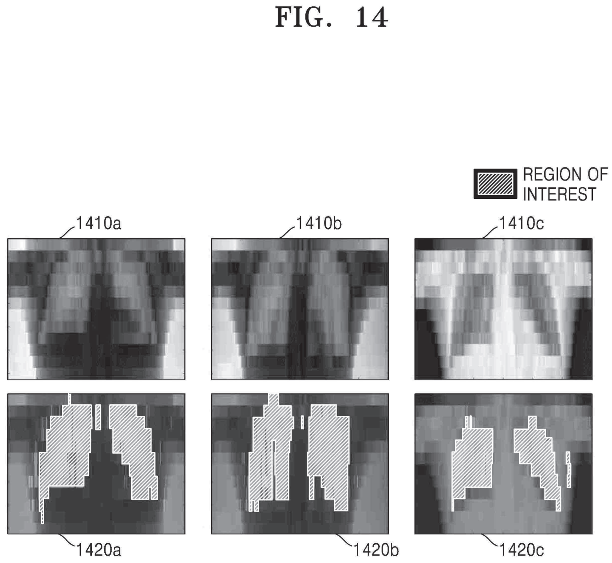

FIG. 14 is a diagram illustrating an example in which an X-ray apparatus determines a region of interest, according to an embodiment of the present disclosure.

**In** an embodiment of the present disclosure, the X-ray apparatus 100 determines a region of interest from scout imaging data 1410a, 1410b and 1410c. In FIG. 14, 1420a is an image defining a region of interest from the scout imaging data 1410a, 1420b is an image defining a region of interest from the scout imaging data 1410b, and 1420c is an image defining a region of interest from the scout imaging data 1410c.

The X-ray apparatus 100 determines the regions of interest from the scout imaging data 1410a, 1410b and 1410c based on the imaging protocol or imaging area selected by the user. The X-ray apparatus 100 may determine an area corresponding to the imaging protocol or imaging area as the region of interest. For example, when an imaging protocol for capturing an image of lungs is selected, the X-ray apparatus 100 may determine an area corresponding to the lungs as the region of interest. Furthermore, for example, when an imaging protocol for capturing an image of bones is selected, the X-ray apparatus 100 may determine an area corresponding to the bones as the region of interest.

In an embodiment of the present disclosure, the X-ray apparatus 100 may determine whether each pixel of the scout imaging data belongs to the region of interest and define pixels corresponding to the region of interest.

In an embodiment of the present disclosure, the X-ray apparatus 100 determines the second exposure time based on pixel values of the region of interest from the scout imaging data. In an embodiment of the present disclosure, the X-ray apparatus 100 may determine the second exposure time based on Equation 1:

【Equation 1】

$$t_{main} = \frac{PV_{target}}{PV_{scout}} \times Coefficient$$

In Equation 1, $t_{main}$ denotes the second exposure time of main imaging, $PV_{scout}$ denotes a pixel value of the region of interest in the scout imaging data, $PV_{target}$ denotes a target value of the pixel value of the region of interest in the main imaging image, and Coefficient denotes a certain constant. In an embodiment of the present disclosure, $PV_{scout}$ and $PV_{target}$ may each be computed as an average of pixel values of the region of interest. The second exposure time $t_{main}$ may be defined as a value in msec. $PV_{target}$ may be determined based on a target dose, the imaging area or the medical institute. Coefficient may be determined based on the attributes of the X-ray detector 200. The target dose may be determined based on the imaging area or the medical institute. The attributes of the X-ray detector 200 may correspond to at least one of sensitivity, a dynamic range or a sampling pixel layout.

In an embodiment of the present disclosure, the second exposure time $t_{main}$ may be determined based on Equation 2:

【Equation 2】

$$t_{main} = \frac{Target\ dose \times sensitivity}{PV_{scout}} \times f \times t_{scout}$$

In Equation 2, 'Target dose' refers to a target cumulative dose of the region of interest, and 'sensitivity' refers to sensitivity of the X-ray detector 200. 'Target doses' may be represented as a uGy value. 'Sensitivity' of the X-ray detector 200 may be represented in the unit of 1/uGy, and is a pre-stored value. Furthermore, f is a pre-stored value that corresponds to an AEC scout calibration factor of the X-ray detector 200. $t_{scout}$ refers to the first exposure time of scout imaging.

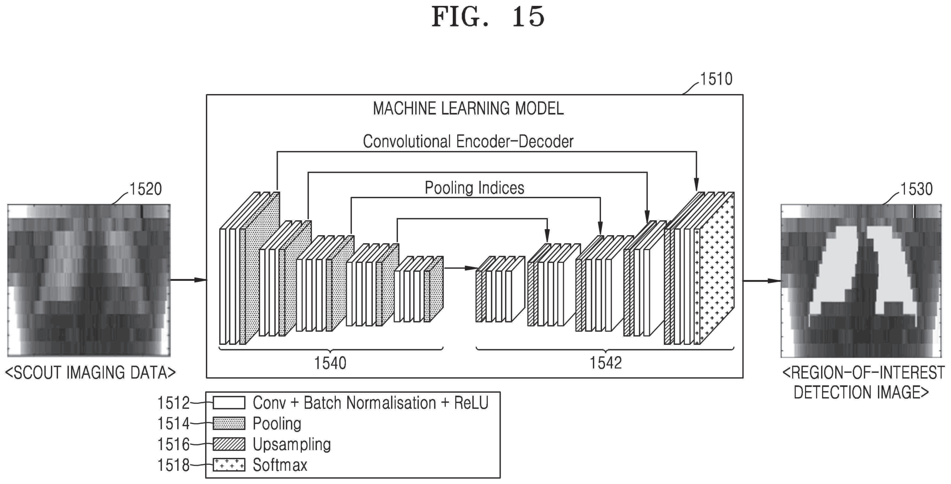

FIG. 15 is a diagram illustrating a procedure for determining a region of interest by using a machine learning model, according to an embodiment of the present disclosure.

In an embodiment of the present disclosure, the X-ray apparatus 100 may determine a region of interest from scout imaging data 1520 by using a machine learning model 1510. The machine learning model 1510 receives the scout imaging data 1520 and outputs a region-of-interest detection image 1530 that defines a region of interest. The region-of-interest detection image 1530 refers to pixels corresponding to the region of interest.

In an embodiment of the disclosure, the machine learning model 1510 corresponds to a segmentation model that identifies a region of interest. The machine learning model 1510 may have a convolution neural network (CNN) structure. As shown in FIG. 15, the machine learning model 1510 includes a plurality of convolution layers 1512 that perform convolution processing, batch normalisation processing, and rectified linear unit (ReLU) processing. Furthermore, the machine learning model 1510 includes a plurality of pooling layers 1514, a plurality of upsampling layers 1516 and a softmax layer 1518. A first group layer 1540 including the plurality of convolution layers 1512 and the plurality of pooling layers 1514 and a second group layer 1542 including the plurality of convolution layers 1512 and the plurality of upsampling layers 1516 correspond to an encoder-decoder structure. The pooling layers 1514 of the first group layer 1540 and the upsampling layers 1516 of the second group layer 1542 make up pooling indices.

In an embodiment of the present disclosure, the machine learning model 1510 may be executed by the processor 120 of the X-ray apparatus 100. The processor 120 may execute instructions of the machine learning model 1510 stored in the memory 410 to perform an operation of the machine learning model 1510.

In an embodiment of the present disclosure, the machine learning model 1510 may be executed by the external device 1020. The X-ray apparatus 100 transmits the scout imaging data 1520 to the external device 1020, and the external device 1020 receives the scout imaging data 1520 from the X-ray apparatus 100. The external device 1020 inputs the received scout imaging data 1520 to the machine learning model 1510 to generate the region-of-interest detection image 1530. In an embodiment of the present disclosure, the external device 1020 transmits the region-of-interest detection image 1530 to the X-ray apparatus 100. Furthermore, in an embodiment of the present disclosure, the external device 1020 computes the second exposure time based on the region-of-interest detection image 1530 and the scout imaging data 1520, and transmits the computed second exposure time to the X-ray apparatus 100.

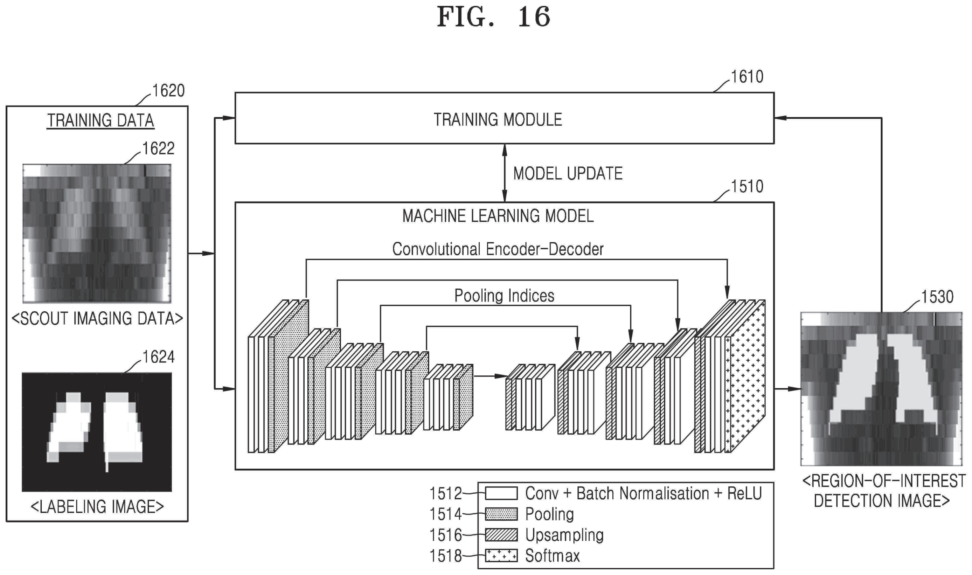

FIG. 16 is a diagram illustrating a procedure for training a machine learning model, according to an embodiment of the present disclosure.

In an embodiment of the present disclosure, the machine learning model 1510 is a model that is machine-trained based on training data 160.

The training data 1620 includes plenty of scout imaging data 1622 and sets of labeling images 1624. The labeling image 1624 is an image defining pixels corresponding to the region of interest in the scout imaging data 1622.

A training module 1610 is a module for training the machine learning model 1510 by using the training data 1620. The training module 1610 inputs the scout imaging data 1622 of the training data 1620 to the machine learning model 1510, and obtains the region-of-interest detection image 1530 output from the machine learning model 1510. The training module 1610 updates the machine learning model 1510 by comparing the region-of-interest detection image 1530 corresponding to the scout imaging data 1622 input to the machine learning model 1510 with the labeling image 1624. The training module 1610 trains the machine learning model 1510 by using a lot of training data 1620.

The machine learning model 1510 may be pre-trained in a stage of designing the X-ray apparatus 100. The training module 1610 may be included in the X-ray apparatus 100, an external device, an external server, or the like.

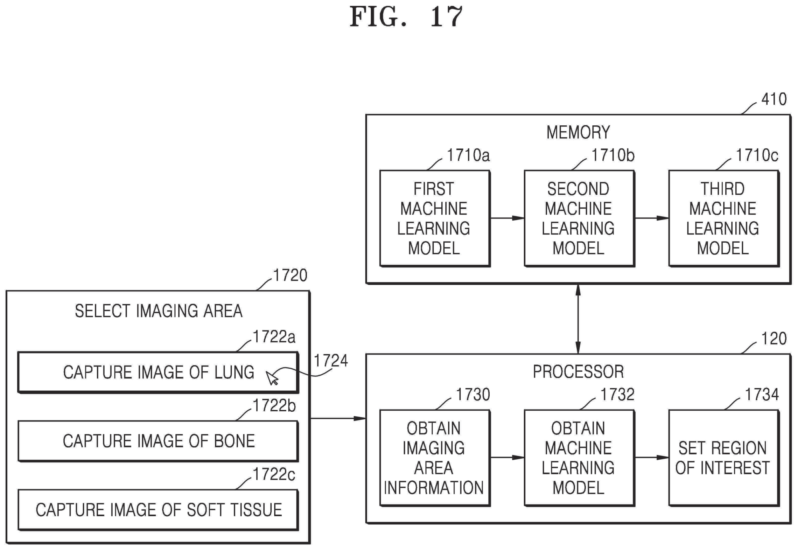

FIG. 17 is a diagram illustrating a procedure for selecting a machine learning model depending on an imaging area, according to an embodiment of the present disclosure.

In an embodiment of the present disclosure, the X-ray apparatus 100 may select one of a plurality of machine learning models 1710a, 1710b, and 1710c according to the imaging protocol or imaging area. The plurality of machine learning models 1710a, 1710b and 1710c correspond different imaging protocols or different imaging areas. The plurality of machine learning models 1710a, 1710b and 1710c select different areas as the region of interest. For example, the first machine learning model 1710a selects lungs as a region of interest, the second machine learning model 1710b selects bones as a region of interest, and the third machine learning model 1710c selects some soft tissues as a region of interest. The plurality of machine learning models 1710a, 1710b and 1710c are machine-trained with different training data depending on the target region of interest. Various types and numbers of the plurality of machine learning models 1710a, 1710b and 1710c may be selected.

The plurality of machine learning models 1710a, 1710b and 1710c may be stored in the X-ray apparatus 100, the external device 1020 or an external server. The processor 120 may select one of the plurality of machine learning models 1710a, 1710b and 1710c based on the imaging protocol or imaging area selected by the user, and use it to determine the region of interest.

The X-ray apparatus 100 receives a user input to select at least one of the imaging protocol or the imaging area before X-ray imaging. The X-ray apparatus 100 may provide a first GUI 1720 to select an imaging protocol or an imaging area. The first GUI 1720 provides selection options 1722a, 1722b and 1722c corresponding to imaging protocols or imaging areas that may be selected by the user. FIG. 17 shows an example of the selection options, and the selection options 1722a, 1722b and 1722c may be implemented in various forms and types. For example, the selection options 1722a, 1722b and 1722c may include the lung imaging option 1722a, the bone imaging option 1722b and the soft tissue imaging option 1722c.

The X-ray apparatus 100 may receive a user input to select in 1724 one of the selection options of the first GUI 1720 through the input interface 1310. The processor 120 receives a user input to select one of the selection options.

The processor 120 obtains imaging area information based on the user input in operation 1730. The processor 120 selects and obtains a machine learning model corresponding to the imaging area information from among the plurality of machine learning models 1710a, 1710b and 1710c based on the imaging area information, in operation 1732. For example, the processor 120 executes the first machine learning model 1710a to set the lungs to the region of interest when the user selects the lung imaging option 1722a. The processor 120 then obtains the region-of-interest detection image by inputting the scout imaging data to the selected machine learning model and sets the region of interest, in operation 1734.

When the plurality of machine learning models 1710a, 1710b and 1710c are stored in the external device or the server, the processor 120 may obtain a machine learning model corresponding to the imaging protocol or imaging area from the external device or the server.

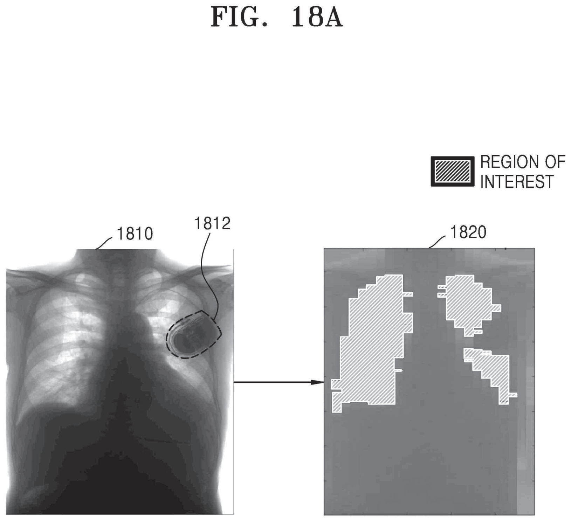

FIG. 18A is a diagram illustrating a procedure for setting a region of interest excluding an artificial device area, according to an embodiment of the present disclosure.

In an embodiment of the disclosure, in determining a region of interest from scout imaging data 1810, the X-ray apparatus 100 determines the region of interest excluding an artificial device 1812. When the user has the artificial device 1812 in his/her body, the artificial device 1812 may be shown in the X-ray image. The artificial device 1812 may correspond to, for example, a pacemaker, an artificial heart, an artificial lung or an artificial joint.

The X-ray apparatus 100 detects the artificial device 1812 from the scout imaging data 1810. When the artificial device 1812 is imaged in the scout imaging data 1810, the X-ray apparatus 100 generates the region-of-interest detection image 1820 that defines a region of interest, excluding an artificial device area corresponding to the artificial device 1812.

In an embodiment of the present disclosure, the X-ray apparatus 100 may detect the artificial device 1812 by using a certain object recognition algorithm. The X-ray apparatus 100 may detect the artificial device 1812 by using X-ray attenuation characteristics, the shape of the artificial device 1812, etc.

In an embodiment of the present disclosure, the X-ray apparatus 100 may detect the artificial device 1812 by using the machine learning model 1510. The machine learning model 1510 may be trained to set a region of interest, excluding an area 1822 corresponding to the artificial device 1812. The machine learning model 1510 may output the region-of-interest detection image 1820 that sets a region of interest excluding the area 1822 corresponding to the artificial device 1812 from the scout imaging data 1810.

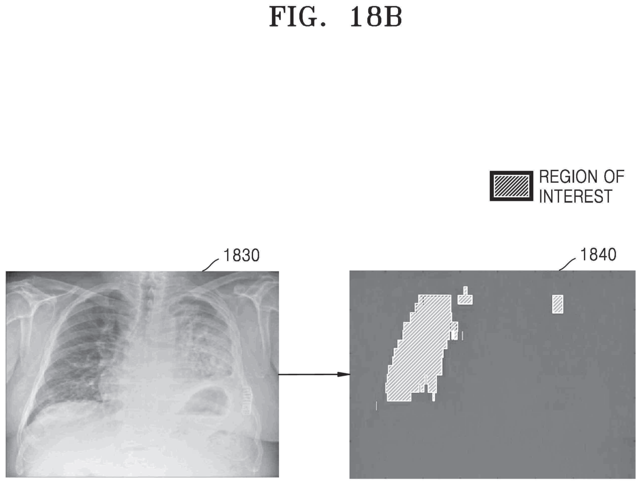

FIG. 18B is a diagram illustrating a procedure for setting a region of interest excluding organ failure regions, according to an embodiment of the present disclosure.

In an embodiment of the present disclosure, the X-ray apparatus 100 may set a region of interest excluding an organ damage area. The organ damage area may include, for example, an organ fibrosis area or an organ resection area, without being limited thereto. The organ damage area may be defined at various organs such as lungs, liver, stomach, heart, etc.

The scout imaging data 1830 is imaging data of a patient with advanced pulmonary fibrosis. The X-ray apparatus 100 may determine a region of interest, excluding the organ damage area with the advanced pulmonary fibrosis. In the scout imaging data 1830, shown are the left lung with advanced pulmonary fibrosis and the region-of-interest detection image 1840 representing a region of interest excluding an area with the advanced pulmonary fibrosis of the left lung.

In an embodiment of the present disclosure, the machine learning model 1510 may set a region of interest excluding the organ damage area. The machine learning model 1510 may be trained with training data with the organ damage area left out of the region of interest.

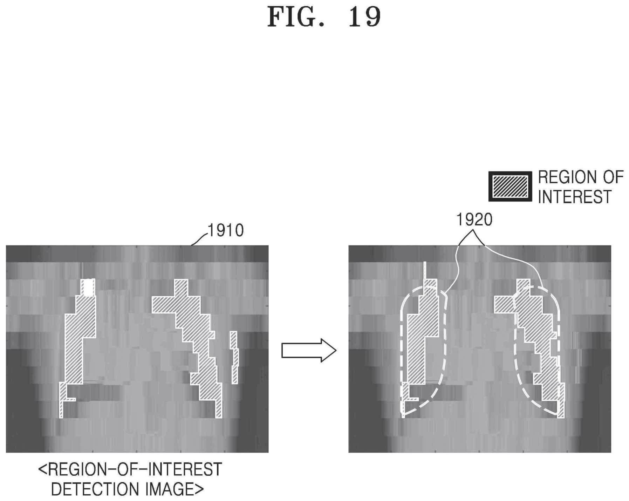

FIG. 19 is a diagram illustrating a case of setting a region of interest to a default area, according to an embodiment of the present disclosure.

In an embodiment of the disclosure, the X-ray apparatus 100 may set a region of interest to a certain default area 1920 when the number of pixels of the region of interest in the region-of-interest detection image 1910 is less than a reference value. **In an** embodiment of the disclosure, when the number of pixels of the region of interest is less than the reference value, reliability of the scout imaging data is deemed to be low, so the region of interest may be set to the certain default area 1920. For example, the number of pixels of the region of interest may be less than the reference value when an organ in the imaging area is damaged (e.g., pulmonary fibrosis), when imaging quality is low, when an artificial device is imaged as well, etc.

The default area 1920 may be differently determined depending on the imaging area. For example, in a case of imaging lungs, the default area 1920 may have a position, shape and size corresponding to the lungs. The default area 1920 may be differently defined depending on the imaging area. The X-ray apparatus 100 may define the default area 1920 based on the imaging protocol or imaging area information.

When the region of interest is set to the default area 1920, the X-ray apparatus 100 may determine the second exposure time based on pixel values of pixels corresponding to the default area 1920.

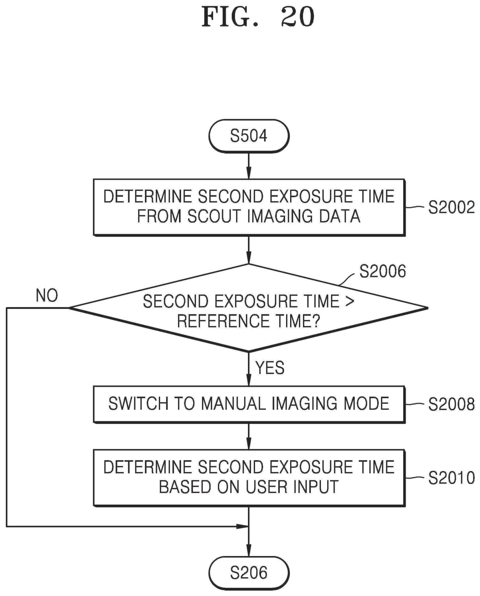

FIG. 20 is a flowchart illustrating a procedure for switching to a manual imaging mode when a second exposure time exceeds a reference time, according to an embodiment of the present disclosure.

In an embodiment of the disclosure, when the second exposure time exceeds the reference time, the X-ray apparatus 100 may be switched to a manual imaging mode.

When the scout imaging data is generated in operation S504, the X-ray apparatus 100 determines the second exposure time from the scout imaging data in operation S2002. As described above, the X-ray apparatus 100 may set a region of interest from the scout imaging data, and determine the second exposure time based on the pixel value of the region of interest, the target pixel value and the Coefficient value.

Next, in operation S2004, the X-ray apparatus 100 determines whether the second exposure time exceeds the reference time. When the second exposure time exceeds the reference time, the X-ray apparatus 100 is switched to the manual imaging mode in operation S2006. The manual imaging mode is a mode in which user manually determines the exposure time of the main imaging.

In an embodiment of the present disclosure, when switching to the manual imaging mode, the X-ray apparatus 100 may output a notification that switching to the manual imaging mode is done. The X-ray apparatus 100 may output the notification that switching to the manual imaging mode is done, through the workstation 180, the output interface of the mobile X-ray apparatus, the external device 1030, etc. Furthermore, when switching to the manual imaging mode, the X-ray apparatus 100 may additionally receive an imaging control signal for main imaging.

Next, in operation S2008, the X-ray apparatus 100 determines the second exposure time based on a user input.

In an embodiment of the present disclosure, in the manual imaging mode, the X-ray apparatus 100 may irradiate X-rays from the X-ray irradiation module 110 while the user is pressing the hand switch. In the manual imaging mode, the X-ray device 100 stops irradiating the X-rays when the user released the pressure on the hand switch. In operation S506, the X-ray apparatus 100 may irradiate X-rays to perform main imaging while the user is pressing the hand switch.

Furthermore, in an embodiment of the present disclosure, in the manual imaging mode, the X-ray apparatus 100 receives a user input to set the second exposure time through the input interface 1310. In operation S506, the X-ray apparatus 100 sets the second exposure time based on a user input and performs main imaging.

In operation S2004, when the second exposure time exceeds the reference time, the X-ray apparatus 100 performs main imaging in operation S506 based on the second exposure time set in operation S2002. When the second exposure time does not exceed the reference time in operation S2004, the X-ray apparatus 100 automatically performs main imaging in operation S506 without the need to receive an additional imaging control signal.

The machine-readable storage medium may be provided in the form of a non-transitory storage medium. The term 'non-transitory storage medium' may mean a tangible device without including a signal, e.g., electromagnetic waves, and may not distinguish between storing data in the storage medium semi-permanently and temporarily. For example, the non-transitory storage medium may include a buffer that temporarily stores data.

In an embodiment of the disclosure, the aforementioned method according to the various embodiments of the disclosure may be provided in a computer program product. The computer program product may be a commercial product that may be traded between a seller and a buyer. The computer program product may be distributed in the form of a machine-readable storage medium (e.g., a CD-ROM) or distributed directly between two user devices (e.g., smart phones) or online (e.g., downloaded or uploaded). In the case of the online distribution, at least part of the computer program product (e.g., a downloadable app) may be at least temporarily stored or arbitrarily created in a storage medium that may be readable to a device such as a server of the manufacturer, a server of the application store, or a relay server.

According to an embodiment of the present disclosure, provided is an X-ray apparatus 100 including an X-ray irradiation module 110 configured to output an X-ray; an X-ray detector 200 configured to detect the X-ray output from the X-ray irradiation module; memory 410 storing at least one instruction; and at least one processor 120 configured to execute the at least one instruction, wherein the at least one processor 120 is configured to execute the at least one instruction to control the X-ray irradiation module 110 to output an X-ray for a first exposure time to perform scout imaging, read out X-ray detection values from a plurality of sampling pixels 610 corresponding to some of a plurality of pixels 612 of the X-ray detector 200 for the first exposure time to generate scout imaging data corresponding to the scout imaging, control the X-ray irradiation module 110 to output an X-ray for a second exposure time determined based on the scout imaging data to perform main imaging, and read out X-ray detection values from the plurality of pixels 612 of the X-ray detector 200 for the second exposure time to generate a main imaging image.

According to an embodiment of the present disclosure, the at least one processor 120 is configured to execute the at least one instruction to determine a region of interest from the scout imaging data, and determine the second exposure time based on pixel values of the region of interest.

According to an embodiment of the present disclosure, the X-ray apparatus 100 may further include an input interface 1310 for receiving a user input, and the at least one processor 120 is configured to execute the at least one instruction to select one of a plurality of machine learning models trained for each imaging area, based on imaging protocol or imaging area information selected by a user through the input interface 1310, obtain region-of-interest information output from the selected machine learning model by inputting the scout imaging data to the selected machine learning model, and determine the region of interest based on the obtained region-of-interest information.

According to an embodiment of the present disclosure, the at least one processor 120 is configured to execute the at least one instruction to identify an artificial device from the scout imaging data, and determine the region of interest excluding the identified artificial device.

According to an embodiment of the present disclosure, the at least one processor 120 is configured to execute the at least one instruction to identify an organ damage area from the scout imaging data, and determine the region of interest excluding the identified organ damage area.

According to an embodiment of the present disclosure, the at least one processor 120 is configured to execute the at least one instruction to determine a preset default area as the region of interest based on the number of pixels of the region of interest determined from the scout imaging data being less than a reference value.

According to an embodiment of the present disclosure, the at least one processor 120 is configured to execute the at least one instruction to determine the second exposure time from the scout imaging data based on pixel values of the region of interest, a target dose and attributes of the X-ray detector 200.

According to an embodiment of the present disclosure, the attributes of the X-ray detector 200 may correspond to at least one of sensitivity, a dynamic range or a sampling pixel layout.

According to an embodiment of the present disclosure, the target dose may be determined based on at least one of an imaging protocol, an imaging area, or a medical institute.

According to an embodiment of the present disclosure, the at least one processor 120 is configured to execute the at least one instruction to determine at least one of an output voltage, an output current, or an output charge quantity of the X-ray irradiation module 110 based on the scout imaging data.

According to an embodiment of the present disclosure, the at least one processor 120 is configured to execute the at least one instruction to, when the determined second exposure time exceeds a reference time, operate in a manual imaging mode for determining the second exposure time according to a user input.

According to an embodiment of the present disclosure, the X-ray apparatus 100 may further include a communication module for communicating with an external device, and the at least one processor 120 is configured to execute the at least one instruction to, through the communication module 1010, transmit the scout imaging data to the external device and receive the second exposure time computed by the external device.

According to an aspect of an embodiment of the present disclosure, the X-ray apparatus 100 may further include a communication module 1310 for communicating with an external device, and the at least one processor 120 is configured to execute the at least one instruction to refresh the X-ray detector 200 after performing the scout imaging, wirelessly transmit the scout image to an external device through the communication module 1310 while performing the refreshing, receive the second exposure time information determined by the external device through the communication module 1310, and set the X-ray irradiation module 110 with the set second exposure time and operation parameters for the main imaging.

According to an aspect of an embodiment of the disclosure, provided is a method of controlling an X-ray apparatus including performing scout imaging by outputting an X-ray for a first exposure time; reading out X-ray detection values from a plurality of sampling pixels corresponding to some of a plurality of pixels of the X-ray detector for the first exposure time to generate scout imaging data corresponding to the scout imaging; performing main imaging by outputting an X-ray for a second exposure time determined based on the scout imaging data; and reading out an X-ray detection value from the plurality of pixels of the X-ray detector for the second exposure time to generate a main imaging image.

According to an embodiment of the present disclosure, a method of controlling an X-ray apparatus may further include determining a region of interest from the scout imaging data; and determining the second exposure time based on pixel values of the region of interest.

According to an embodiment of the present disclosure, a method of controlling an X-ray apparatus may further include selecting one of a plurality of machine learning models trained for each imaging area, based on imaging protocol or imaging area information selected by a user input; obtaining region-of-interest information output from the selected machine learning model by inputting the scout imaging data to the selected machine learning model; and determining the region of interest based on the obtained region-of-interest information.

According to an embodiment of the present disclosure, a method of controlling an X-ray apparatus may further include identifying an artificial device from the scout imaging data; and determining the region of interest excluding the identified artificial device.

According to an embodiment of the present disclosure, a method of controlling an X-ray apparatus may further include determining a preset default area as the region of interest based on the number of pixels of the region of interest determined from the scout imaging data being less than a reference value.

According to an embodiment of the present disclosure, a method of controlling an X-ray apparatus may further include determining the second exposure time from the scout imaging data based on pixel values of the region of interest, a target dose and attributes of the X-ray detector.

According to an embodiment of the disclosure, provided is a computer-readable recording medium having a program recorded thereon to cause a computer to perform an X-ray apparatus control method.

**Claims**

1. An X-ray apparatus (100) comprising:

   an X-ray irradiation module (110) configured to output an X-ray;
   an X-ray detector (200) configured to detect the X-ray output from the X-ray irradiation module;
   memory (410) storing at least one instruction; and
   at least one processor (120) configured to execute the at least one instruction,
   wherein the at least one processor (120) is configured to execute the at least one instruction to
   control the X-ray irradiation module (110) to output an X-ray for a first exposure time to perform scout imaging,
   read out X-ray detection values from a plurality of sampling pixels (610) corresponding to some of a plurality of pixels (612) of the X-ray detector (200) for the first exposure time to generate scout imaging data corresponding to the scout imaging,
   control the X-ray irradiation module (110) to output an X-ray for a second exposure time determined based on the scout imaging data to perform main imaging, and
   read out X-ray detection values from the plurality of pixels (612) of the X-ray detector (200) for the second exposure time to generate a main imaging image.

2. The X-ray apparatus (100) of claim 1, wherein the at least one processor (120) is configured to execute the at least one instruction to

   determine a region of interest from the scout imaging data, and
   determine the second exposure time based on pixel values of the region of interest.

3. The X-ray apparatus (100) of claim 2, further comprising:

   an input interface (1310) configured to receive a user input,
   wherein the at least one processor (120) is configured to execute the at least one instruction to
   select one of a plurality of machine learning models trained for each imaging area, based on imaging protocol or imaging area information selected by a user through the input interface (1310),
   obtain region-of-interest information output from the selected machine learning model by inputting the scout imaging data to the selected machine learning model, and
   determine the region of interest based on the obtained region-of-interest information.

4. The X-ray apparatus (100) of claim 2, wherein the at least one processor (120) is configured to execute the at least one instruction to identify an artificial device from the scout imaging data, and
   determine the region of interest excluding the identified artificial device.

5. The X-ray apparatus (100) of claim 2, wherein the at least one processor (120) is configured to execute the at least one instruction to identify an organ damage area from the scout imaging data, and
   determine the region of interest excluding the identified organ damage area.

6. The X-ray apparatus (100) of claim 2, wherein the at least one processor (120) is configured to execute the at least one instruction to determine a preset default area as the region of interest based on the number of pixels of the region of interest determined from the scout imaging data being less than a reference value.

7. The X-ray apparatus (100) of any one of claims 1 to 6, wherein the at least one processor (120) is configured to execute the at least one instruction to determine the second exposure time from the scout imaging data based on pixel values of the region of interest, a target dose and attributes of the X-ray detector 200.

8. The X-ray apparatus (100) of claim 7, wherein the attributes of the X-ray detector (200) correspond to at least one of sensitivity, a dynamic range or a sampling pixel layout.

**9.** The X-ray apparatus (100) of claim 7, wherein the target dose is determined based on at least one of an imaging protocol, an imaging area or a medical institute.

**10.** The X-ray apparatus (100) of any one of claims 1 to 9, wherein the at least one processor (120) is configured to execute the at least one instruction to determine at least one of an output voltage, an output current, or an output charge quantity of the X-ray irradiation module (110) based on the scout imaging data.

**11.** The X-ray apparatus (100) of any one of claims 1 to 10, wherein the at least one processor (120) is configured to execute the at least one instruction to, when the determined second exposure time exceeds a reference time, operate in a manual imaging mode for determining the second exposure time according to a user input.

**12.** The X-ray apparatus (100) of any one of claims 1 to 11, further comprising:

a communication module configured to communicate with an external device,
wherein the at least one processor (120) is configured to execute the at least one instruction to
transmit the scout imaging data to the external device through the communication module (1010), and
receive the second exposure time computed by the external device.

**13.** The X-ray apparatus (120) of any one of claims 1 to 12, further comprising:

a communication module (1310) configured to communicate with an external device,
wherein the at least one processor (120) is configured to execute the at least one instruction to
refresh the X-ray detector (200) after performing the scout imaging,
while performing the refreshing,
wirelessly transmit the scout image to an external device through the communication module (1310),
receive the second exposure time information determined by the external device through the communication module (1310), and
set the X-ray irradiation module (110) with a set second exposure time and operation parameters for the main imaging.

**14.** A method of controlling an X-ray apparatus, the method comprising:

performing scout imaging by outputting an X-ray for a first exposure time;
reading out X-ray detection values for the first exposure time from a plurality of sampling pixels corresponding to some of a plurality of pixels of the X-ray detector and generating scout imaging data corresponding to the scout imaging;
performing main imaging by outputting an X-ray for a second exposure time determined based on the scout imaging data; and
reading out X-ray detection values from the plurality of pixels of the X-ray detector for the second exposure time to generate a main imaging image.

**15.** A computer-readable recording medium having a program recorded thereon to cause a computer to perform the method of claim 14.

# FIG. 1

# FIG. 2

# FIG. 3

100

110

103

151

152

200

105

101

120 — PROCESSOR

140 — COMMUNICATION MODULE

310

SERVER

320

MEDICAL DEVICE

330

PORTABLE TERMINAL

# FIG. 4

# FIG. 5

```
        ( START )
            │
            ▼
┌─────────────────────────┐
│  PERFORM SCOUT IMAGING   │── S502
│   FOR FIRST EXPOSURE     │
│          TIME            │
└─────────────────────────┘
            │
            ▼
┌─────────────────────────┐
│ GENERATE SCOUT IMAGING   │── S504
│          DATA            │
└─────────────────────────┘
            │
            ▼
┌─────────────────────────┐
│   PERFORM MAIN IMAGING   │── S506
│  FOR SECOND EXPOSURE     │
│   TIME DETERMINED        │
│ BASED ON SCOUT IMAGING   │
│          DATA            │
└─────────────────────────┘
            │
            ▼
┌─────────────────────────┐
│ GENERATE MAIN IMAGING    │── S508
│          IMAGE           │
└─────────────────────────┘
            │
            ▼
         ( END )
```

FIG. 6

# FIG. 7

# FIG. 8

EP 4 706 543 A1

SCOUT IMAGING

MAIN IMAGING

110 — X-RAY IRRADIATION MODULE

200 — X-RAY DETECTOR

802

Scout-Shot — 808

Main-Shot — 816

| Ready | Window Time | Readout | Refresh | Window Time | Readout |
|---|---|---|---|---|---|
| 804 | 806 | 810 | 812 | 814 | 818 |

# FIG. 9

X-RAY IRRADIATION MODULE — 110

X-RAY DETECTOR — 200

WORKSTATION — 180

IRRADIATE X-RAY FOR FIRST EXPOSURE TIME — S902

READ OUT SAMPLING PIXEL — S904

COMPUTE SECOND EXPOSURE TIME FROM SCOUT IMAGING DATA — S906

CONTROL X-RAY IRRADIATION MODULE — S908

IRRADIATE X-RAY FOR SECOND EXPOSURE TIME — S910

READ OUT PIXEL — S912

TRANSMIT MAIN IMAGING IMAGE — S914

DISPLAY MAIN IMAGING IMAGE — S916

EP 4 706 543 A1

# FIG. 10

# FIG. 11

X-RAY IRRADIATION MODULE — 110

X-RAY DETECTOR — 200

EXTERNAL DEVICE — 180

S902 — IRRADIATE X-RAY FOR FIRST EXPOSURE TIME

READ OUT SAMPLING PIXEL — S904

TRANSMIT SCOUT IMAGING DATA — S1102

COMPUTE SECOND EXPOSURE TIME FROM SCOUT IMAGING DATA — S1104

COMPUTE AND TRANSMIT SECOND EXPOSURE TIME — S1106

S910 — IRRADIATE X-RAY FOR SECOND EXPOSURE TIME

READ OUT PIXEL — S912

TRANSMIT MAIN IMAGING IMAGE — S914

DISPLAY MAIN IMAGING IMAGE — S916

# FIG. 12

FIG. 13

# FIG. 14

# FIG. 15

<SCOUT IMAGING DATA> 1520

MACHINE LEARNING MODEL 1510

Convolutional Encoder-Decoder

Pooling Indices

1540   1542

<REGION-OF-INTEREST DETECTION IMAGE> 1530

1512 — Conv + Batch Normalisation + ReLU
1514 — Pooling
1516 — Upsampling
1518 — Softmax

EP 4 706 543 A1

# FIG. 16

TRAINING DATA — 1620

<SCOUT IMAGING DATA> — 1622

<LABELING IMAGE> — 1624

TRAINING MODULE — 1610

MODEL UPDATE

MACHINE LEARNING MODEL — 1510

Convolutional Encoder-Decoder

Pooling Indices

1512 — Conv + Batch Normalisation + ReLU
1514 — Pooling
1516 — Upsampling
1518 — Softmax

<REGION-OF-INTEREST DETECTION IMAGE> — 1530

EP 4 706 543 A1

# FIG. 17

# FIG. 18A

# FIG. 18B

# FIG. 19

REGION OF INTEREST

1910

1920

<REGION-OF-INTEREST DETECTION IMAGE>

# FIG. 20

```
        ┌─────────┐
        │  S504   │
        └────┬────┘
             ↓
┌─────────────────────────────────┐
│ DETERMINE SECOND EXPOSURE TIME  │──── S2002
│    FROM SCOUT IMAGING DATA      │
└─────────────────┬───────────────┘
                  ↓
                                      S2006
          ╱─────────────────────╲
  NO ─────┤  SECOND EXPOSURE TIME > ├
          │    REFERENCE TIME?      │
          ╲─────────────────────╱
  │               │ YES
  │               ↓
  │   ┌─────────────────────────────┐
  │   │ SWITCH TO MANUAL IMAGING MODE│──── S2008
  │   └───────────────┬─────────────┘
  │                   ↓
  │   ┌─────────────────────────────┐
  │   │ DETERMINE SECOND EXPOSURE TIME│──── S2010
  │   │    BASED ON USER INPUT       │
  │   └───────────────┬─────────────┘
  │                   │
  └───────────────────┤
                      ↓
                ┌─────────┐
                │  S206   │
                └─────────┘
```

## EP 4 706 543 A1

## INTERNATIONAL SEARCH REPORT

International application No.

**PCT/KR2024/005700**

| A. | CLASSIFICATION OF SUBJECT MATTER |
|---|---|

**A61B 6/00**(2006.01)i; **G16H 40/60**(2018.01)i; **G06N 20/00**(2019.01)i; **A61B 6/42**(2024.01)i; **A61B 6/58**(2024.01)i

According to International Patent Classification (IPC) or to both national classification and IPC

| B. | FIELDS SEARCHED |
|---|---|

Minimum documentation searched (classification system followed by classification symbols)

A61B 6/00(2006.01); A61B 6/08(2006.01); A61B 6/10(2006.01); G01T 1/17(2006.01); G01T 1/24(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 엑스레이 (x-ray), 노출 (exposure), 시간 (time), 픽셀 (pixel), 샘플링 (sampling), 디텍터 (detector), 관심영역 (ROI)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2013-138828 A (FUJIFILM CORP.) 18 July 2013 (2013-07-18)<br>See paragraphs [0031], [0037], [0039] and [0065]-[0117]; claim 18; and figure 1. | 1,2,4-9,14,15 |
| A | | 3 |
| A | KR 10-2015-0107335 A (RAYENCE CO., LTD.) 23 September 2015 (2015-09-23)<br>See claims 1-7. | 1-9,14,15 |
| A | JP 2016-131754 A (SHIMADZU CORP.) 25 July 2016 (2016-07-25)<br>See claims 1-10. | 1-9,14,15 |
| A | KR 10-2013-0057996 A (CARESTREAM HEALTH, INC.) 03 June 2013 (2013-06-03)<br>See entire document. | 1-9,14,15 |
| A | JP 2020-514740 A (GENERAL ELECTRIC COMPANY) 21 May 2020 (2020-05-21)<br>See entire document. | 1-9,14,15 |

☐ Further documents are listed in the continuation of Box C.　　☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **01 August 2024** | **02 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2024/005700** |

**Box No. II    Observations where certain claims were found unsearchable (Continuation of item 2 of first sheet)**

This international search report has not been established in respect of certain claims under Article 17(2)(a) for the following reasons:

1. ☐  Claims Nos.:
   because they relate to subject matter not required to be searched by this Authority, namely:

2. ☐  Claims Nos.:
   because they relate to parts of the international application that do not comply with the prescribed requirements to such an extent that no meaningful international search can be carried out, specifically:

3. ☑  Claims Nos.: **10-13**
   because they are dependent claims and are not drafted in accordance with the second and third sentences of Rule 6.4(a).

Form PCT/ISA/210 (continuation of first sheet) (July 2022)

INTERNATIONAL SEARCH REPORT
Information on patent family members

International application No.

**PCT/KR2024/005700**

| Patent document cited in search report | | | Publication date (day/month/year) | Patent family member(s) | | | Publication date (day/month/year) |
|---|---|---|---|---|---|---|---|
| JP | 2013-138828 | A | 18 July 2013 | CN | 103156627 | A | 19 June 2013 |
| | | | | CN | 103156627 | B | 03 August 2016 |
| | | | | JP | 5602198 | B2 | 08 October 2014 |
| | | | | US | 2013-0148782 | A1 | 13 June 2013 |
| KR | 10-2015-0107335 | A | 23 September 2015 | KR | 10-1606746 | B1 | 28 March 2016 |
| | | | | US | 10188366 | B2 | 29 January 2019 |
| | | | | US | 2017-0000445 | A1 | 05 January 2017 |
| | | | | WO | 2015-137759 | A1 | 17 September 2015 |
| JP | 2016-131754 | A | 25 July 2016 | JP | 6716196 | B2 | 01 July 2020 |
| KR | 10-2013-0057996 | A | 03 June 2013 | CN | 102834053 | A | 19 December 2012 |
| | | | | CN | 102834053 | B | 25 November 2015 |
| | | | | CN | 102834054 | A | 19 December 2012 |
| | | | | CN | 102858246 | A | 02 January 2013 |
| | | | | CN | 102858246 | B | 28 January 2015 |
| | | | | CN | 102917646 | A | 06 February 2013 |
| | | | | CN | 102917646 | B | 21 October 2015 |
| | | | | CN | 102934526 | A | 13 February 2013 |
| | | | | CN | 105662446 | A | 15 June 2016 |
| | | | | CN | 105662446 | B | 27 March 2020 |
| | | | | CN | 106852114 | A | 13 June 2017 |
| | | | | EP | 2557992 | A2 | 20 February 2013 |
| | | | | EP | 2557992 | A4 | 09 April 2014 |
| | | | | EP | 2557992 | B1 | 01 March 2017 |
| | | | | EP | 2557993 | A2 | 20 February 2013 |
| | | | | EP | 2557993 | A4 | 02 April 2014 |
| | | | | EP | 2557994 | A2 | 20 February 2013 |
| | | | | EP | 2557994 | A4 | 02 April 2014 |
| | | | | EP | 2557994 | B1 | 05 August 2015 |
| | | | | EP | 2557997 | A2 | 20 February 2013 |
| | | | | EP | 2557997 | B1 | 05 August 2015 |
| | | | | EP | 2559325 | A2 | 20 February 2013 |
| | | | | EP | 2559325 | B1 | 24 October 2018 |
| | | | | EP | 3209208 | A1 | 30 August 2017 |
| | | | | EP | 3209208 | B1 | 04 December 2019 |
| | | | | ES | 2547144 | T3 | 02 October 2015 |
| | | | | JP | 2013-523396 | A | 17 June 2013 |
| | | | | JP | 2013-523397 | A | 17 June 2013 |
| | | | | JP | 2013-523398 | A | 17 June 2013 |
| | | | | JP | 2013-523400 | A | 17 June 2013 |
| | | | | JP | 2013-524477 | A | 17 June 2013 |
| | | | | JP | 2016-139619 | A | 04 August 2016 |
| | | | | JP | 5658352 | B2 | 21 January 2015 |
| | | | | JP | 5833632 | B2 | 16 December 2015 |
| | | | | JP | 6231148 | B2 | 15 November 2017 |
| | | | | KR | 10-1770858 | B1 | 23 August 2017 |
| | | | | KR | 10-1777436 | B1 | 11 September 2017 |
| | | | | KR | 10-2013-0057977 | A | 03 June 2013 |
| | | | | KR | 10-2013-0057978 | A | 03 June 2013 |
| | | | | KR | 10-2013-0057991 | A | 03 June 2013 |
| | | | | US | 10165992 | B2 | 01 January 2019 |

Form PCT/ISA/210 (patent family annex) (July 2022)

# EP 4 706 543 A1

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| | | | | US | 10285656 | B2 | 14 May 2019 |
| | | | | US | 2011-0249791 | A1 | 13 October 2011 |
| | | | | US | 2011-0249792 | A1 | 13 October 2011 |
| | | | | US | 2011-0249793 | A1 | 13 October 2011 |
| | | | | US | 2011-0249799 | A1 | 13 October 2011 |
| | | | | US | 2011-0249804 | A1 | 13 October 2011 |
| | | | | US | 2011-0249805 | A1 | 13 October 2011 |
| | | | | US | 2011-0249806 | A1 | 13 October 2011 |
| | | | | US | 2011-0249807 | A1 | 13 October 2011 |
| | | | | US | 2012-0039447 | A1 | 16 February 2012 |
| | | | | US | 2014-0341349 | A1 | 20 November 2014 |
| | | | | US | 2016-0174918 | A1 | 23 June 2016 |
| | | | | US | 2016-0181053 | A1 | 23 June 2016 |
| | | | | US | 2017-0303882 | A1 | 26 October 2017 |
| | | | | US | 8568028 | B2 | 29 October 2013 |
| | | | | US | 8672543 | B2 | 18 March 2014 |
| | | | | US | 8821017 | B2 | 02 September 2014 |
| | | | | US | 8824634 | B2 | 02 September 2014 |
| | | | | US | 8827554 | B2 | 09 September 2014 |
| | | | | US | 8867705 | B2 | 21 October 2014 |
| | | | | US | 8873712 | B2 | 28 October 2014 |
| | | | | US | 8876379 | B2 | 04 November 2014 |
| | | | | US | 9155509 | B2 | 13 October 2015 |
| | | | | WO | 2011-130198 | A2 | 20 October 2011 |
| | | | | WO | 2011-130198 | A3 | 19 January 2012 |
| | | | | WO | 2011-130203 | A2 | 20 October 2011 |
| | | | | WO | 2011-130203 | A3 | 23 February 2012 |
| | | | | WO | 2011-130207 | A2 | 20 October 2011 |
| | | | | WO | 2011-130207 | A3 | 08 March 2012 |
| | | | | WO | 2011-130210 | A2 | 20 October 2011 |
| | | | | WO | 2011-130210 | A3 | 16 February 2012 |
| | | | | WO | 2011-130214 | A2 | 20 October 2011 |
| | | | | WO | 2011-130214 | A3 | 23 February 2012 |
| | | | | WO | 2016-064993 | A1 | 28 April 2016 |
| JP | 2020-514740 | A | 21 May 2020 | CN | 110520760 | A | 29 November 2019 |
| | | | | EP | 3596511 | A1 | 22 January 2020 |
| | | | | EP | 3596511 | B1 | 15 June 2022 |
| | | | | JP | 7053650 | B2 | 12 April 2022 |
| | | | | US | 10222489 | B2 | 05 March 2019 |
| | | | | US | 2018-0259657 | A1 | 13 September 2018 |
| | | | | WO | 2018-169595 | A1 | 20 September 2018 |

Form PCT/ISA/210 (patent family annex) (July 2022)